# EUROPEAN PATENT SPECIFICATION

(11) **EP 1 216 251 B1**
(45) Date of publication and mention of the grant of the patent: **26.02.2003**
(21) Application number: 00966904.5
(22) Date of filing: 27.09.2000
(51) Int. Cl.: C07F 7/18, C07D 498/18

(54) **REGIOSELECTIVE SYNTHESIS OF RAPAMYCIN DERIVATIVES**
REGIOSELEKTIVE SYNTHESE VON RAPAMYCINDERIVATEN
SYNTHESE STEREOSELECTIVE DE DERIVES DE RAPAMYCINE

(30) Priority: 29.09.1999 US 408830
(43) Date of publication of application: 26.06.2002
(73) Proprietor: Wyeth, Madison, New Jersey 07940-0874 (US)
(72) Inventor: SHAW, Chia-Cheng, Ville Saint Laurent, Quebec H4L 3R5 (CA); SELLSTEDT, John, Hamilton, Lachine, Quebec H8T 1P7 (CA); NOURELDIN, Razzak, Brossard, Quebec J4W 2X6 (CA); CHEAL, Gloria, Karen, Beaconsfield, Quebec H9W 4R6 (CA); FORTIER, Genevieve, Westmount, Quebec H3Z 1M2 (CA)
(74) Representative: Wileman, David Francis, Dr.
(86) International application number: US0026445
(87) International publication number: WO01023395

(56) References cited:
- US-A- 5 362 718

## Description

### BACKGROUND OF THE INVENTION

This invention relates to the regioselective synthesis of derivatives of rapamycin at the 42-position, which are useful for inducing immunosuppression, and in the treatment of transplantation rejection, graft vs. host disease, autoimmune diseases, diseases of inflammation, adult T-cell leukemia/lymphoma, solid tumors, fungal infections, and hyperproliferative vascular disorders. More particularly this invention provides an essentially selective process for preparing 31-silyl protected ethers of rapamycin useful as intermediates to 42-esters and ethers of rapamycin.

Rapamycin is a macrocyclic triene antibiotic produced by Streptomyces hygroscopicus, which was found to have antifungal activity, particularly against Candida albicans, both in vitro and in vivo [C. Vezina et al., J. Antibiot. 28, 721 (1975); S.N. Sehgal et al., J. Antibiot. 28, 727 (1975); H. A. Baker et al., J. Antibiot. 31, 539 (1978); U.S. Patent 3,929,992; and U.S. Patent 3,993,749].

Rapamycin alone (U.S. Patent 4,885,171) or in combination with picibanil (U.S. Patent 4,401,653) has been shown to have antitumor activity. R. Martel et al. [Can. J. Physiol. Pharmacol. 55, 48 (1977)] disclosed that rapamycin is effective in the experimental allergic encephalomyelitis model, a model for multiple sclerosis; in the adjuvant arthritis model, a model for rheumatoid arthritis; and effectively inhibited the formation of IgE-like antibodies.

The immunosuppressive effects of rapamycin have been disclosed in FASEB 3, 3411 (1989). Cyclosporin A and FK-506, other macrocyclic molecules, also have been shown to be effective as immunosuppressive agents, therefore useful in preventing transplant rejection [FASEB 3, 3411 (1989); FASEB 3, 5256 (1989); R.Y. Calne et al., Lancet 1183 (1978); and U.S. Patent 5,100,899].

Rapamycin has also been shown to be useful in preventing or treating systemic lupus erythematosus [U.S. Patent 5,078,999], pulmonary inflammation [U.S. Patent 5,080,899], insulin dependent diabetes mellitus [U.S. Patent 5,321,009], smooth muscle cell proliferation and intimal thickening following vascular injury [U.S. Patent 5,516,781], adult T-cell leukemia/lymphoma [European Patent Application 525,960 A1], and ocular inflammation [U.S. Patent 5,387,589].

Numerous rapamycin 42-derivatives are known, typically being esters (carbon and sulfur based) or ethers of the 42-hydroxyl group of rapamycin, that are produced by esterification or etherification of the 42-position. Esterification of rapamycin at the 42-position was commonly prepared by directly reacting rapamycin with acylating agents in order to afford the desired product. The chemistry appeared to be rather simple. However, as rapamycin contains two secondary hydroxyl groups at positions 31 and 42, attempts to discriminate between these two functional centers in order to achieve a selective synthesis of 42-monoacylated product, posed a difficult challenge. This type of non-regioselective reaction also produced a 31,42-bis-acylated by-product and as well, some unreacted rapamycin remained in the reaction mixture. The final result was a lower yield that required extensive purification to obtain pure 42-monoacylated product. The problems can be illustrated by reference to the synthesis of 42-monoester such as rapamycin 42-ester with 2,2-bis-(hydroxymethyl)propionic acid (referred herein as compound [C]). For example, the synthesis of rapamycin 42-ester with 2,2-bis-(hydroxymethyl)propionic acid described in US patent 5,362,718, example 10, was non-regioselective, the 31,42-bisester by-product was also generated. As a result, the crude product [B] after work-up contains the desired product [B], 31,42-bisester by-product and unreacted rapamycin. In an effort to consume the remaining starting rapamycin, the reaction was allowed to proceed for a longer period with negative consequences, the quantity of the 31,42-bisester increased significantly. The resulting crude product [B] is contaminated with unreacted rapamycin and 31,42-bisester, and subsequent column chromatography purification effort has proved to be difficult as the 42,31-bisester has a very close retention time with product [B]. Overall, the major obstacle in large-scale production of compound [B] appears to be the non-regiospecificity that is further complicated by purification difficulties.

There is therefore a need for a regioselective synthesis of 42-esters or ethers of rapamycin.

There is a need for a process for selectively preparing 42-esters or ethers of rapamycin that is suitable for commercial or large scale production and which can be carried out efficiently.

### DESCRIPTION OF THE INVENTION

In a first aspect this invention provides a regioselective process for preparing a 31-silyl ether of rapamycin, eg a compound of formula IA 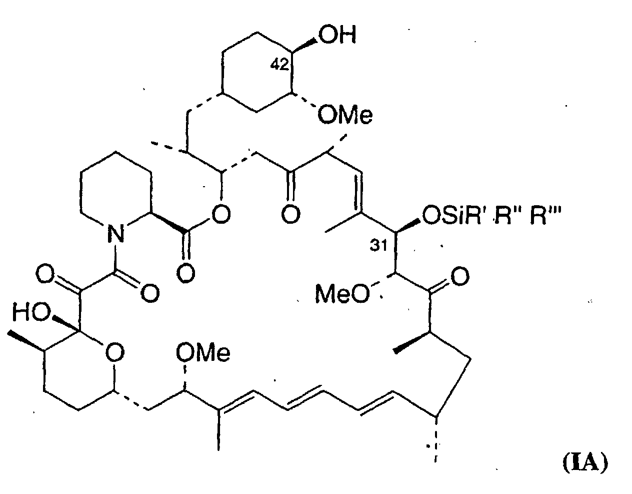 wherein R', R" and R"' are each organic groups such as alkyl groups preferably containing 1 to 6 carbon atoms, eg 1 to 4 carbon atoms most preferably methyl, ethyl and propyl, which comprises:
(a) treating rapamycin with a silylating agent to form rapamycin 31,42-bis-silyl ether; and
(b) hydrolysing the 42-silyl ether group in cold dilute acid to provide rapamycin 31-silyl ether, eg a compound of formula (I) above.

The rapamycin 31-silyl ether may be acylated or etherified on the 42-hydroxy group followed by removal of the 31-silyl protecting group and any other protecting group that might be present to give desired 42-esters or ethers of rapamycin.

Accordingly in a second aspect this invention provides a regioselective method for the preparation of a 42-ester or ether rapamycin having the structure (I) 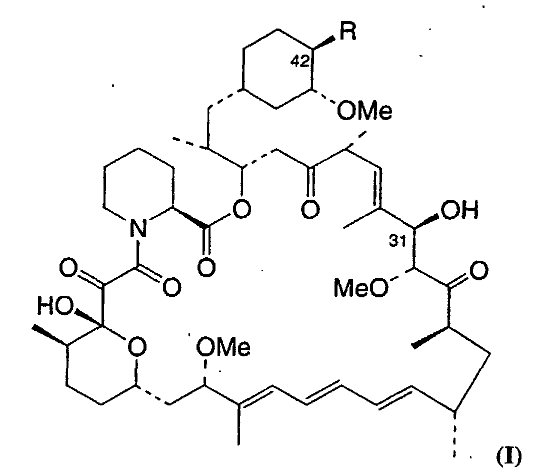 wherein R is an ester or ether, which comprises:
(a) treating rapamycin with a silylating agent to form rapamycin 31,42-bis-silyl ether;
(b) hydrolysing the 42-silyl ether in cold dilute acid to provide rapamycin 31-silyl ether;
(c) treating the rapamycin 31-silyl ether with a suitable esterifying or etherifying reagent to form rapamycin 31-silyl ether 42-ester or ether; and
(d) hydrolysing the 31-silyl ether in cold dilute acid and if desired removing any protecting group on R simultaneously or sequentially to provide the desired rapamycin 42-ester or ether.

Preferred 42-esters and ethers of rapamycin which can be prepared by the method provided by this invention are disclosed in the following patents, which are all hereby incorporated by reference: alkyl esters (U.S. Patent 4,316,885); aminoalkyl esters (U.S. Patent 4,650,803); fluorinated esters (U.S. Patent 5,100,883); amide esters (U.S. Patent 5,118,677); carbamate esters (U.S. Patent 5,118,678); silyl ethers (U.S. Patent 5,120,842); aminoesters (U.S. Patent 5,130,307); acetals (U.S. Patent 5,51,413); aminodiesters (U.S. Patent 5,162,333); sulfonate and sulfate esters (U.S. Patent 5,177,203); esters (U.S. Patent 5,221,670); alkoxyesters (U.S. Patent 5,233,036); O-aryl, -alkyl, -alkenyl, and -alkynyl ethers (U.S. Patent 5,258,389); carbonate esters (U.S. Patent 5,260,300); arylcarbonyl and alkoxycarbonyl carbamates (U.S. Patent 5,262,423); carbamates (U.S. Patent 5,302,584); hydroxyesters (U.S. Patent 5,362,718; WO 95/28406); hindered esters (U.S. Patent 5,385,908); heterocyclic esters (U.S. Patent 5,385,909); gem-disubstituted esters (U.S. Patent 5,385,910); amino alkanoic esters (U.S. Patent 5,389,639); phosphorylcarbamate esters (U.S. Patent 5,391,730); carbamate esters (U.S. Patent 5,411,967); carbamate esters (U.S. Patent 5,434,260); amidino carbamate esters (U.S. Patent 5,463,048); carbamate esters (U.S. Patent 5,480,988); carbamate esters (U.S. Patent 5,480,989); carbamate esters (U.S. Patent 5,489,680); hindered N-oxide esters (U.S. Patent 5,491,231); biotin esters (U.S. Patent 5,504,091); and O-alkyl ethers (U.S. Patent 5,665,772). These patents also disclose various values for the equivalent of R in formula (I) above and methods for esterification or etherification utilized in step (c), above.

Particularly preferred values for R in formula (I) above are
(a) -O-C=O.CR⁷R⁸R⁹; (ie., esters) wherein:
   - R⁷: is hydrogen, alkyl of 1-6 carbon atoms, alkenyl of 2-7 carbon atoms, alkynyl of 2-7 carbon atoms, -(CR³R⁴)_{f}OR¹⁰, -CF₃, -F, or -CO₂R¹¹;
   - R⁸ and R⁹: are each, independently, hydrogen, alkyl of 1-6 carbon atoms, or -(CR³R⁴)_{f}OR¹⁰; or R⁸ and R⁹ may be taken together to form X;
   - R¹⁰: is hydrogen, alkyl of 1-6 carbon atoms, alkenyl of 2-7 carbon atoms, alkynyl of 2-7 carbon atoms, triphenylmethyl, benzyl, alkoxymethyl of 2-7 carbon atoms, chloroethyl, or tetrahydropyranyl;
   - X: is 5-(2,2-di-(alkyl of 1-6 carbon atoms))[1,3]dioxanyl, 5-(2-spiro(cycloalkyl of 3-8 carbon atoms))[1,3]dioxanyl, 4-(2,2-di-(alkyl of 1-6 carbon atoms))[1,3]-dioxanyl, 4-(2-spiro(cycloalkyl of 3-8 carbon atoms))[1,3]dioxanyl, 4-(2,2-di-(alkyl of 1-6 carbon atoms))[1,3]dioxalanyl, or 4-(2-spiro(cycloalkyl of 3-8 carbon atoms))[1,3]dioxalanyl;
   - R³ and R⁴: are each, independently, hydrogen, alkyl of 1-6 carbon atoms, alkenyl of 2-7 carbon atoms, alkynyl of 2-7 carbon atoms, trifluoromethyl, or -F;
   and
   f = 0-6; with the proviso that R contains at least one -(CR³R⁴)_{f}OR¹⁰ or X group; and
(b) - OR¹ (ie., ethers) where R¹ is alkyl, thioalkyl, arylalkyl, hydroxyalkyl, dihydroxyalkyl, hydroxyalkylarylalkyl, dihydroxyalkylarylalkyl, alkoxyalkyl, acyloxyalkyl, aminoalkyl, alkylaminoalkyl, alkoxycarbonylaminoalkyl, acylaminoalkyl, arylsulfonamidoalkyl, allyl, dihydroxyalkylallyl, and dioxolanylallyl, carbalkoxyalkyl, wherein "alk-" or "alkyl" refers to C₁₋₆alkyl, branched or linear, preferably C₁₋₃ alkyl, in which the carbon chain may be optionally interrupted by an ether (-O-) linkage; "acyl" represents alkylcarbonyl and "aryl" has 6-10 carbon atoms and includes phenyl, naphthyl and the like.

Most preferably R¹ is selected from hydroxyalkyl, hydroxyalkoxyalkyl, acylaminoalkyl, and aminoalkyl; especially 40-O-(2-hydroxy)ethyl-rapamycin, 40-O-(3-hydroxy)propyl-rapamycin, 40-O-[2-(2-hydroxy)ethoxy]ethyl-rapamycin, and 40-O-(2-acetaminoethyl)-rapamycin).

Most preferably R in formula I is 2,2-bis(hydroxymethyl)propionyloxy or 2,2,5-trimethyl[1,3-dioxane-5-carbonyloxy.

With regard to step (a) the silylation may be carried out in an inert solvent, e.g ethyl acetate; preferably in the presence of a suitable base, e.g. imidazole. The reaction may be carried out at low temperature, eg room temperature or below eg 0°C; preferably 0-5°C. It is preferred that the 31-, and 42-hydroxyl groups are protected as trialkyl silyl ethers. The 42-silyl protected hydroxyl group of the 31, 42-bis-silylated rapamycin can be selectively cleaved under mildly acidic conditions to provide 31-silyl rapamycin. The silylating agents used for this transformation are common, commercially available chloroalkylsilanes, such as chlorotrimethylsilane, chlorotriethylsilane or chlorotripropylsilane. However, the bulkier the trialkylsilane, the more time is needed to deprotect in acid media during the penultimate chemical step to regenerate the 31-hydroxyl group. Also, a longer reaction time in the acid media generates more degradation by-products. Although chlorotrimethylsilane, chloro-triethylsilane or chlorotripropylsilane can be used for the preparation of rapamycin 31-O-trialkylsilyl ethers, chlorotrimethylsilane is the preferred silylating agent. The trimethylsilyl group is more acid labile and therefore easier to de-protect during the transformation and in effect, this minimizes the formation of degradation products. In a preferred process rapamycin is treated with excess chlorotrimethylsilane in ethyl acetate at 0 - 5°C in the presence of an organic base and the 42- and 31- hydroxyl groups of rapamycin are silylated to form rapamycin 31,42-bis-O-trimethylsilyl ether in quantitative yield. The common organic bases such as imidazole, 1-methylimidazole, triethylamine and N, N-diisopropylethylamine can be used for the general silylation reaction. However, imidazole is found to be the preferred base for the silylation of rapamycin as the reaction can be completed within 30 minutes.

With regard to step (b) we have surprisingly found that de-protection to remove the 42-silyl ether group of rapamycin 31,42-bis-O-silyl ether to form rapamycin 31-O-silyl ether may be carried out in cold dilute acid essentially quantitatively producing only very small amounts of by products such as rapamycin (ie the product of complete deprotection) eg less than 20% but generally less than 10% relative to 31 silyl ether. As is shown herein the product of the deprotection step (b) can comprise as much as ∼80% or more of the 31-silyl ether protected rapamycin and less than ∼10% rapamycin with the possibility of rapamycin levels being reduced to as low as ∼1%. This selective deprotection is conveniently carried out using dilute organic or inorganic acid, especially inorganic acids such as sulfuric, hydrochloric or phosphoric, e.g <2.5N, preferably 0.8N to about 2.5N, most preferably 0.1N to 1N. Sulfuric acid is particularly preferred. Preferably the reaction is carried out in a two phase aqueous acid /organic solvent system e.g using ethyl acetate as the second phase, particularly where trimethylsilyl is used as the protecting group which can be selectively removed in for example about 2 to 3 hours. Desirably low reaction temperatures e.g about 25°C or below, preferably about 15°C or below are used, such as from about -5°C to + 10°C, most preferably from 0 to 5°C. Most preferably the de-protection is effected after the silylation reaction *in situ* at 0 - 5°C with ethanol, ethanol-water mixtures, water and dilute inorganic or organic acids. Sulfuric acid (0.5 N) is particularly preferred since the reaction is clean and can be completed in 2 - 5 hours which is convenient for commercial production. However longer reaction times have been found necessary where less reactive silyl protecting groups are employed, eg tri-ethylsilyl or tri-propylsilyl and in such cases a single phase solvent system can be used to selectively de-protect, eg acid/acetone.

A number of organic solvents can be used for silylation and in particular, DMF is often mentioned in the literature. However, in this invention, ethyl acetate is the preferred solvent for step (a) so that a two phase reaction medium is used for the subsequent deprotection step (b).

With regard to step (c) the esterification or etherification of the 31-protected rapamycin can be carried out under conditions described in the patents listed above. For example, in Scheme I shown hereinafter, the acylation of rapamycin 31-trimethylsilyl ether was accomplished using 2,4,6-trichlorobenzoyl mixed anhydride of 2,2,5-trimethyl[1.3-dioxane]-5-carboxylic acid in the presence of 4-dimethylaminopyridine or a similar reagent. In addition, 2,2,5-trimethyl[1.3-dioxane]-5-carboxylic acid chloride was also found to be an effective acylation agent in this invention in the presence of 4-dimethylaminopyridine or a similar reagent. For the acylation conditions, methylene chloride is the preferred solvent rather than tetrahydrofuran which was described in the prior art. The reaction may be carried out at a temperature of from about -50°C to about +25°C. However, lower reaction temperatures of less than 0°C, with -20 to -15°C or lower being more preferred, provide better results than the room temperature acylation described in US Patent 5,362,718.

With regard to step (d) the removal of the 31 silyl protecting group from the 42-esterified or etherified-31 silyl rapamycin may be effected by hydrolysing eg using dilute acid such as described above, preferably a dilute inorganic acid such as sulfuric, hydrochloric or phosphoric acid. Depending on whether it is desired to remove other protecting groups simultaneously the acid may be about 0.1N to about 3N; preferably from about 0.2N to about 2N, most preferably about 0.5N. Conveniently step (d) is carried out in a single phase aqueous acid/organic solvent system, eg. where the organic solvent is acetone. The reaction may be carried out at a temperature about 25°C or below, eg. from about -5°C to about 10°C, preferably from about 0°C to about 5°C.

In the following Scheme I, the acylation products, 31-O-TMS, 42-(protected-hydroxy) esters (compound [E]) can be further treated with diluted acid to convert them to 42-(protected-hydroxy) esters (compound [B]) or used directly to make final product 42-hydroxyesters (final product [C]). This methodology can be used to prepare other esters or ethers of rapamycin, by simply varying the esterfiying or etherifying agent used.

The following scheme also illustrates the regioselective preparation of rapamycin 42-ester with 2,2-bis-(hydroxymethyl)propionic acid, as a representative 42-ester of rapamycin, which can be prepared according to the method provided in this invention. The original synthesis of rapamycin 42-ester with 2,2-bis-(hydroxymethyl)propionic acid is disclosed in US Patent 5,362,718.

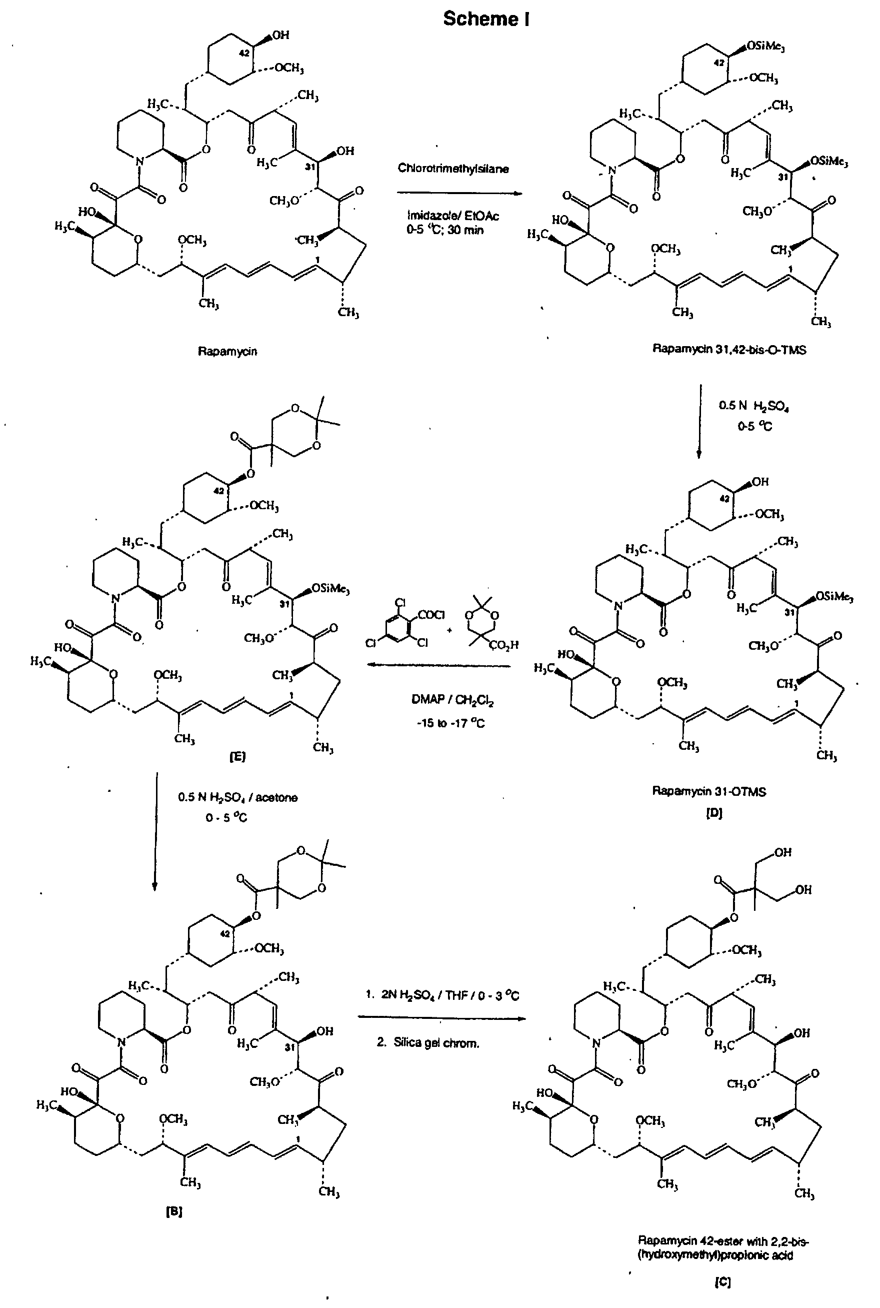

In Scheme I, conversion of compound [B] to rapamycin 42-ester with 2,2-bis-(hydroxymethyl)propionic acid [C], can be accomplished under mildly acidic conditions. It is preferred that aqueous sulfuric acid is used, as it minimizes the formation of impurities generated when aqueous hydrochloric acid is used, as described in the US Patent 5,362,718. The tetraene impurity formed when using hydrochloric acid has been reported to be difficult to separate from the desired product by column chromatography (Caufield *et al*, Tetrahedron Lett., 1994, 37, 6835). It is also preferable to carry out the hydrolysis at 0 - 5°C rather than room temperature as described in US Patent 5,362,718.

Accordingly this invention also provides a process for preparing a compound of formula (C): 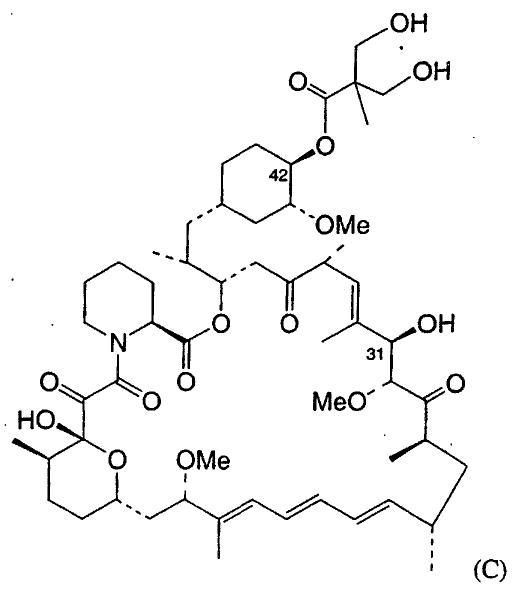 which comprises hydrolysing a compound of formula (B): 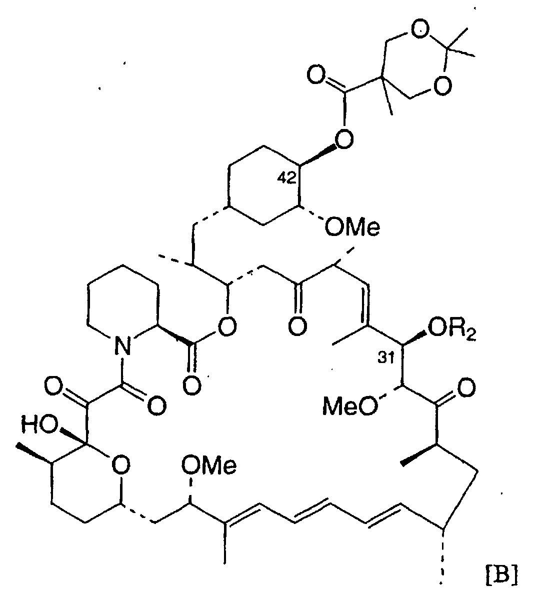 in which R₂ is hydrogen or -SiR'R"R"' wherein R', R" and R"' are the same or different selected from alkyl of 1-6 carbon atoms, phenyl and benzyl; using dilute sulfuric acid, eg using 1N to 3N sulfuric acid. Preferably the reaction is carried out at a temperature of -5°C to +10°C. Tetrahydrofuran is preferably used as solvent.

The synthetic route disclosed in this invention provides several distinct advantages over the synthetic methodology which has been published for the preparation of rapamycin esters or ethers; mainly in the yield and ease of purification of the desired 42-esters or ethers. As this is a regioselective synthesis, the overall yields of the desired 42-esters or ethers is dramatically improved. For example, the synthetic methodology taught in US Patent 5,362,718 provides compound **[B]** in a 35% yield, whereas, the synthesis of **[B]** is accomplished in 85% yield using the methodology disclosed herein. Additionally, the conversion to rapamycin 42-ester with 2,2-bis-(hydroxymethyl)propionic acid from **[B]** is accomplished in approximately 75% yield using the process described herein, whereas only a 20% conversion is provided using the methodology of US Patent 5,362,718.

Using the same methodology, 42-ethers of rapamycin can be prepared in a regioselective manner. As an example, US Patent 5,665,772 discloses the preparation of 40-O-alkyl ethers of rapamycin in a non-regioselective manner. Owing to nomenclature differences, the 42-position of rapamycin (as named in this invention) is referred to as the 40-position in US Patent 5,665,772. These positons are identical. Using the methodology disclosed herein, rapamycin 31-O-trimethylsilyl ether can be treated with, for example, 2-(t-butyldimethylsilyl)oxyethyl triflate) to provide 31-O-trimethylsilyl, 42-O-[2-(t-butyldimethylsilyl)oxy]ethyl-rapamycin. Removal of the silyl protecting groups from the 31-hydroxyl group of rapamycin and from the 42-hydroxyethyl moiety can be accomplished under mildly acidic conditions, such as dilute sulfuric acid to provide 42-O-(2-hydroxy)ethyl rapamycin. The non-regioselective formation of other.42-ethers of rapamycin is disclosed in US Patent 5,665,772. These also can be prepared regioselectively via rapamycin 31-O trimethylsilyl ether.

This invention also covers 31-silyl ethers of rapamycin and 31-silyl ethers of 42-esterified or etherifed derivatives of rapamycin, which are useful in the preparation of the 42-esters and ethers of rapamycin, as disclosed herein. The silicon moiety as represented by -SiR'R"R"', contains 3 groups which can be the same or different. Typical silyl ethers of this invention contain R', R", or R"' moieties which are alkyl of 1-6 carbon atoms, phenyl, or benzyl groups. The alkyl groups can be branched or straight chain. It is preferred that R', R", and R"' be alkyl groups, and more preferred that R', R", and R"' are methyl or ethyl. It is still more preferred that the 31-silyl ether is rapamycin 31-O-trimethylsilyl ether.

Accordingly this invention provides 31-silyl ethers of formula (IA) and (IB): 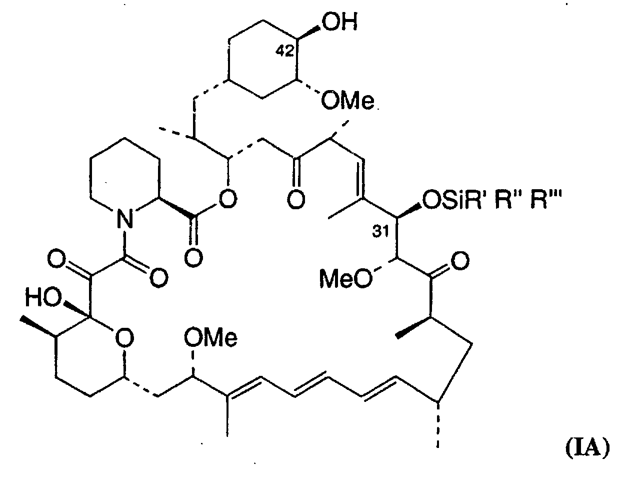 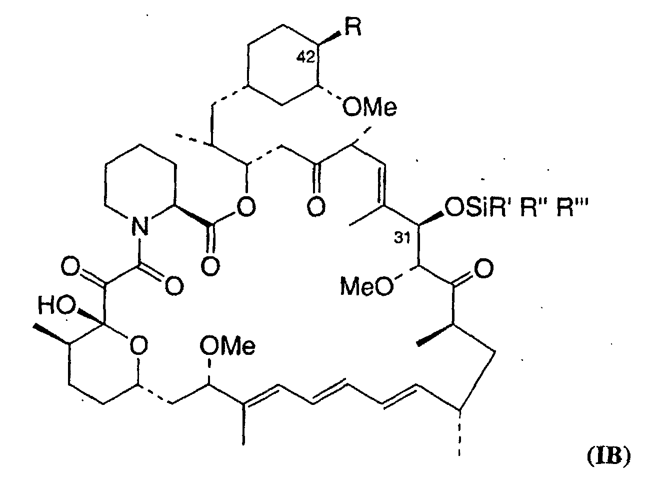 in which formulae,
R is an ester or ether group such as described above and R', R" and R"' are the same or different selected from alkyl of 1-6 carbon atoms, phenyl and benzyl.

The following examples illustrate the preparation of rapamycin 31-silyl ethers and a rapamycin 42-ester, which is representative of the compounds which can be prepared by the processes of this invention.

### EXAMPLE 1

### Rapamycin 31-O-trimethylsilyl ether

A solution of rapamycin (25.0 g, 92.4 % strength; 25.28 mmol) in 750 mL ethyl acetate was cooled to 0 - 5°C; 7.5 g (110.20 mmol) of imidazole was added and stirred to form a solution. To this cold solution 11.0 g (101.25 mmol) of chlorotrimethylsilane was added dropwise over 30 min and stirred for a further 30 min at 0 - 5°C in order to complete the formation of rapamycin 31,42-bis-O-trimethylsilyl ether. A 50 mL quantity of 0.5 N sulfuric acid was added dropwise over a 10 min period and the mixture was stirred for 2.5 h at 0 - 5°C. The reaction mixture was transferred into a separatory funnel and the aqueous layer was separated and extracted with 125 mL ethyl acetate. The organic layers were combined and successively washed with brine (125 mL), saturated sodium bicarbonate solution (100 mL), water (125 mL x 2) then brine to pH 6 - 7. The organic layer was dried over anhydrous sodium sulfate and evaporated under reduced pressure to give a beige color foam product, 28.5 g (theory 24.94 g). HPLC analysis showed it contained 86% (by area %) of rapamycin 31-O-trimethylsilyl ether and 7% of rapamycin. The product was used directly for the subsequent reaction.
LC/MS electrospray (+) mode (M - H) = 985. 'H NMR (400 MHz, d-6 DMSO) δ 4.60 (m, 1H, (42C)OH), 4.10 (m, 1H, C(31) H), 3.09 (m, 1H, C(42) H), -0.027 (s, 9H, 31-O-TMS).

### EXAMPLE 2

### 2,2,5-Trimethyl[1,3-dioxane]-5-carboxylic acid chloride

A solution of 2,2,5-trimethyl[1.3-dioxane]-5-carboxylic acid (17.42 g, 0.1 mol) in 200 mL of dry toluene was warmed to 40°C, and 26.0 mL of oxalyl chloride (37.83 g, 0.3 mol) added dropwise over a period of 30 min and then stirred at 40°C for 2.5 h. The reaction mixture was evaporated under reduced pressure to remove solvent and excess oxalyl chloride. The residual product was evaporated twice with dry toluene (200 mL), then dried under high vacuum at 40°C for 2 h to obtain 19.75 g of product as an orange colored liquid. ¹H NMR (300 MHz, CDCl₃) δ 4.28 (2H, d, J = 10.5Hz), 3.76 (2H, d, J = 10.5 Hz),1.46 (3H, s),1.29 (3H, s). ¹³C NMR (75 MHz, CDCl₃) δ 176.43, 98.76, 66.06, 52.07, 25.82, 21.20, 18.10.

### EXAMPLE 3

### Rapamycin 31-O-trimethylsilyl ether, 42-ester with 2,2,5-trimethyl[1,3-dioxane]-5-carboxylic acid

### Method A:

To a solution of the 2,2,5-trimethyl[1.3-dioxane]-5-carboxylic acid (9.77 g, 56.08 mmol) and N,N-diisopropylethylamine (12.00 g, 92.80 mmol) in 200 mL methylene chloride at room temperature under nitrogen, 2,4,6-trichlorobenzoyl chloride (13.35 g, 54.73 mmol) was added and the resulting mixture was stirred for 5 hours at room temperature. The reaction mixture was cooled to -20 to -15°C and a solution of rapamycin 31-O-trimethylsilyl ether (28.50 g, crude, made from 25.28 mmol of rapamycin) in 120 mL methylene chloride was added. A solution of 4-dimethylaminopyridine (11.68 g, 95.60 mmol) in 110 mL methylene chloride was added dropwise over a 2 h period. The reaction mixture was further stirred for 16 h at -15 to -16°C. The reaction mixture was quenched with 100 mL water and the organic layer was separated and washed with 0.5 N sulfuric acid (180 mL), followed by brine (100 mL), saturated sodium bicarbonate solution (100 mL), water (100 mL x 2), brine (100 mL) to pH 6- 7. The organic layer was dried over anhydrous sodium sulfate and evaporated under reduced pressure to afford the title compound (33.18 g) as a beige color foam.
LC/MS electrospray (+) mode (M + NH₄) = 1160. ¹H NMR (400 MHz, d-6 DMSO) δ 4.57 (m, 1H, C(42)H), 4.10 (m, 1H, C(31) H), 4.03 (d, 2H), 3.57 (d, 2H), 1.34 (s, 3H), 1.24 (s, 3H), 1.13 (s, 3H), -0.023 (s, 9H, 31-O-TMS)

### Method B:

A solution of rapamycin 31-O-trimethylsilyl ether (11.00g; from 10.0 g of rapamycin; 11.15 mmol) in 120 mL methylene chloride, containing 2 mL of N,N,-dimethylformamide, was stirred under nitrogen and cooled to -15°C, 4-dimethylaminopyridine (4.80 g, 39.29 mmol) was added and the mixture was stirred to form a solution. To this cold solution a 7.5% solution of 2,2,5-trimethyl[1.3-dioxane]-5-carboxylic acid chloride (42.18 g; 16.42 mmol) in methylene chloride was added dropwise over a 2 h period. The solution was further stirred for 1 h at -15°C, and an additional 7.5% solution of acid chloride (14.06 g, 5.47 mmol) in methylene chloride was added over a 30 min period. The reaction mixture was further stirred for 16 h at -15°C to -16°C. The reaction mixture was quenched with 100 mL brine and the organic layer was separated and washed with cold 0.5 N sulfuric acid (100 mL), brine, (100 mL), saturated sodium bicarbonate solution (100 mL) water (100 mL), brine (100 mL) to pH 6 - 7. The organic layer was dried over anhydrous sodium sulfate and evaporated under reduced pressure to afford product (12.15 g) as a yellow foam.

### EXAMPLE 4

### Rapamycin 42-ester with 2,2,5-trimethyl[1,3-dioxane]-5-carboxylic acid

A solution of rapamycin 31-O-trimethylsilyl ether, 42-ester with 2,2,5-trimethyl[1.3-dioxane]-5-carboxylic acid (33.18 g; from example 3, method A) in 100 mL of acetone was stirred and cooled to 0 - 5°C. To this cold solution 17 mL of 0.5 N sulfuric acid was added dropwise over a 10 min period and the mixture was stirred for 2.5 h at 0 - 5°C. A solution of sodium bicarbonate (1.44 g) in 20 mL water was added over a period of 20 min followed by an additional 33 mL water over a period of 30 min; the product started to precipitated after about 1 h of stirring. The mixture was stirred overnight at 0 - 5°C and after filtration the solid product was washed with 60 mL of acetone-water (1:1). The product was dried in a vacuum oven at 30°C to obtain 28.85 g of product (83.9% strength, 89.3% overall yield from rapamycin). The ¹H NMR of the product was identical to the product described in US Patent 5,362,718 example 10.

### EXAMPLE 5

### Rapamycin 42-ester with 2,2-bis-(hydroxymethyl)propionic acid

### Method A:

A solution of rapamycin 42-ester with 2,2,5-trimethyl[1.3-dioxane]-5-carboxylic acid (28.85 g; from example 4) in 276 mL of tetrahydrofuran was stirred and cooled to 0 - 5°C. To this cold solution 83 mL of cold 2 N sulfuric acid was added dropwise over a 30 min period and the mixture was stirred for 60 h at 0 - 5°C. The reaction mixture was diluted with 600 mL of ethyl acetate and washed with 120 mL brine. The aqueous layer was extracted once with 120 mL of ethyl acetate and the organic extracts were combined and washed with saturated sodium bicarbonate solution (120 mL), water (200 mL x 2) and brine (120 mL) to pH 6 -7. The organic phase was dried over anhydrous sodium sulfate and evaporated under reduced pressure at room temperature to obtain product (28.42 g), as a beige color foam. The crude product was chromatographed on a silica gel column and eluted with 30% acetone in heptane to give 18.06 g of pure product, a white solid (69.4% overall from rapamycin). The ¹H NMR of the product is identical to the product described in US Patent 5,362,718 example 11.

### Method B:

A solution of rapamycin 31-O-trimethylsilyl ether, 42-ester with 2,2,5-trimethyl[1.3-dioxane]-5-carboxylic acid (23.25 g, prepared from 20.06 g of rapamycin, strength 92.7%, 20.34 mmol) in 230 mL of tetrahydrofuran was stirred and cooled to 0 - 5°C. To this cold solution 115 mL of cold 2 N sulfuric acid was added dropwise over a 45 min period and the mixture was stirred for 88 h at 0 - 5°C. The reaction mixture was diluted with 500 mL of ethyl acetate and washed with 100 mL brine. The aqueous layer was extracted once with 100 mL of ethyl acetate and the organic extracts were combined and washed with saturated sodium bicarbonate solution (80 mL), water (80 mL x2) and brine (100 mL) to pH 6 -7. The organic phase was dried over anhydrous sodium sulfate and evaporated under reduced pressure at room temperature to afford product (22.48 g), a beige color foam. The crude product was chromatographed on a silica gel column and eluted with 30% acetone in heptane to give 16.50 g of pure product as a white solid (78.4% overall from rapamycin). The ¹H NMR of the product is identical to the product described in US Patent 5,362,718 example 11.

### EXAMPLE 6

### Rapamycin 31,42-bis-O-trimethylsilyl ether

A solution of rapamycin (10.0 g, 94.3% strength; 10.3 mmol) in 150 mL ethyl acetate was cooled to 0 - 5°C; 3.0 g (44 mmol) of imidazole was added and stirred to form a solution. To this cold solution, 4.4 g (40.5 mmol) of chforotrimethylsilane was added dropwise over a 20 min period and following this, the solution was stirred at 0 - 5°C for a further 30 min. The reaction mixture was filtered to remove the imidazole HCl and the filtrate was evaporated under reduced pressure to obtain a yellow foam. Heptane (200 mL) was added and stirred at room temperature for 20 min and the mixture was filtered. The filtrate was washed with 40 mL of saturated sodium bicarbonate solution, then twice with water (80 mL), then brine (50 mL). The organic layer was dried over anhydrous sodium sulfate and evaporated to obtain the product, a yellow foam of 11.42 g (98.6%).
LC/MS electrospray (-) mode (M - H) = 1057. ¹H NMR (400 MHz, d-6 DMSO) δ 4.10 (m, 1H, C(31) H), 3.31 (m, 1H, C(42) H), 0.057 (s, 9H, 42-O-TMS), -0.027 (s, 9H,31-O-TMS).

### EXAMPLE 7

### Rapamycin 31-O-triethylsilyl ether

A solution of rapamycin (5.00 g, 92.7% strength; 5.07 mmol) in 75 mL ethyl acetate was cooled to 0 - 5°C; 1.50 g (22.03 mmol) of imidazole was added and stirred to form a solution. To this cold solution, 3.05 g (20.23 mmol) of chlorotriethylsilane was added dropwise over a 10 minutes period. The mixture was stirred for 30 min at 0 - 5°C, then stirred at room temperature overnight to complete the formation of rapamycin 31,42-bis-O-triethylsilyl ether. Following filtration of the reaction mixture, the filtrate was evaporated under reduced pressure at room temperature to remove most of the solvent. The residual solution (ca. 10 mL) was dissolved in 60 mL acetone and 15 mL of 0.15 N sulfuric acid was added and the mixture stirred for 25 h at 0 - 5°C. The rapamycin 31,42-bis-O-triethylsilyl ether disappeared at this stage. The reaction mixture was diluted with 80 mL of ethyl acetate and successively washed with brine (60 mL x 2), saturated sodium bicarbonate solution (40 mL), water (60 mL x 2), brine (60 mL) to pH 6 - 7. The organic layer was dried over anhydrous sodium sulfate and evaporated under reduced pressure to obtain a product of light yellow gum, 6.92 g (theory 5.21 g). HPLC analysis showed it contained 95.2% (by area %) rapamycin 31-O-triethylsilyl ether and 0.9% rapamycin.

### EXAMPLE 8

### Rapamycin 31-O-tripropylsilyl ether

A solution of rapamycin (5.00 g, 92.7% strength; 5.07 mmol) in 75 mL ethyl acetate was cooled to 0 - 5°C; 1.50 g (22.03 mmol) of imidazole was added and stirred to form a solution. To this cold solution, 3.91 g (20.3 mmol) of chlorotripropylsilane was added dropwise over 10 min period. The mixture was stirred for 30 min at 0 - 5°C, then at room temperature for 21 hours to complete the formation of rapamycin 31,42-bis-O-tripropylsilyl ether. Following filtration of the reaction mixture, the filtrate was evaporated under reduced pressure at room temperature to remove most of the solvent and the residual solution was dissolved in 60 mL acetone. A 15 mL quantity of 0.25 N of sulfuric acid was added and the mixture was stirred for 45 h at 0 - 5°C; the rapamycin 31,42-bis-O-tripropylsilyl ether disappeared at this stage. The reaction mixture was diluted with 100 mL of ethyl acetate, and successively washed with brine (40 mL x 2), saturated sodium bicarbonate solution (40 mL), water (40 mL x 2), and brine (50 mL) to pH 6 - 7. The organic layer was dried over anhydrous sodium sulfate and evaporated under reduced pressure to obtain a product of light yellow gum, 8.07 g (theory 5.43 g). HPLC analysis showed it contained 96.7% (by area %) of rapamycin 31-O-tripropylsilyl ether and 1% of rapamycin.

## Claims

1. A process for preparing rapamycin 31-silyl ether, which comprises:
(a) reacting rapamycin with a silylating agent to form rapamycin 31,42-bis-silyl ether; and
(b) hydrolysing the 31,42-bis-silyl ether with cold dilute acid to provide rapamycin 31-silyl ether.

2. A process for preparing a 42-ester or ether of rapamycin having the structure 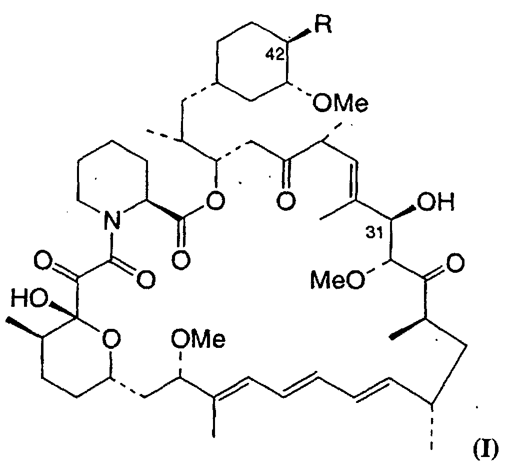 wherein R is an ester or ether, which comprises:
(a) reacting rapamycin with a silylating agent to form rapamycin 31,42-bis-silyl ether;
(b) hydrolysing the 31,42-bis-silyl ether in cold dilute acid to provide rapamycin 31-silyl ether;
(c) reacting the rapamycin 31-silyl ether with a suitable esterifying or etherifying reagent to form rapamycin 31-silyl ether 42-ester or ether; and
(d) hydrolysing the 31-silyl ether in cold dilute acid to provide the desired rapamycin 42-ester or ether; if desired sequentially or simultaneously removing any other protecting group present.

3. A process as claimed in claim 2 in which R in the compound of formula (I) is selected from:
either
(i) -O-C=O.CR⁷R⁸R⁹; (ie., esters) wherein:
R⁷ is hydrogen, alkyl of 1-6 carbon atoms, alkenyl of 2-7 carbon atoms, alkynyl of 2-7 carbon atoms, -(CR³R⁴)_{f}OR¹⁰, -CF₃, -F, or -CO₂R¹¹;
R⁸ and R⁹ are each, independently, hydrogen, alkyl of 1-6 carbon atoms, or- (CR³R⁴)_{f}OR¹⁰; or R⁸ and R⁹ may be taken together to form X;
R¹⁰ is hydrogen, alkyl of 1-6 carbon atoms, alkenyl of 2-7 carbon atoms, alkynyl of 2-7 carbon atoms, triphenylmethyl, benzyl, alkoxymethyl of 2-7 carbon atoms, chloroethyl, or tetrahydropyranyl;
X is 5-(2,2-di-(alkyl of 1-6 carbon atoms))[1,3]dioxanyl, 5-(2-spiro(cycloalkyl of 3-8 carbon atoms))[1,3]dioxanyl, 4-(2,2-di-(alkyl of 1-6 carbon atoms))[1,3]-dioxanyl, 4-(2-spiro(cycloalkyl of 3-8 carbon atoms))[1,3]dioxanyl, 4-(2,2-di-(alkyl of 1-6 carbon atoms))[1,3]dioxalanyl, or 4-(2-spiro(cycloalkyl of 3-8 carbon atoms))[1,3]dioxalanyl;
R³ and R⁴ are each, independently, hydrogen, alkyl of 1-6 carbon atoms, alkenyl of 2-7 carbon atoms, alkynyl of 2-7 carbon atoms, trifluoromethyl, or -F;
and
f = 0-6; with the proviso that R contains at least one -(CR³R⁴)_{f}OR¹⁰ or X group;
or
(ii) -OR¹ (ie., ethers) where R¹ is alkyl, thioalkyl, arylalkyl, hydroxyalkyl, dihydroxyalkyl, hydroxyalkylarylalkyl, dihydroxyalkylarylalkyl, alkoxyalkyl, acyloxyalkyl, aminoalkyl, alkylaminoalkyl, alkoxycarbonylaminoalkyl, acylaminoalkyl, arylsulfonamidoalkyl, allyl, dihydroxyalkylallyl and dioxolanylallyl, carbalkoxyalkyl; wherein "alk-" or "alkyl" refers to C₁₋₆alkyl, branched or linear, preferably C₁₋₃ alkyl, in which the carbon chain may be optionally interrupted by an ether (-O-) linkage; "acyl" represents alkylcarbonyl and "aryl" has 6-10 carbon atoms.

4. A process as claimed in claim 3 in which R¹ is selected from hydroxyalkyl, hydroxyalkoxyalkyl, acylaminoalkyl, and aminoalkyl.

5. A process as claimed in claim 4 in which R¹ is selected from 2-hydroxyethyl; 3-hydroxypropyl; 2-[(2-hydroxy)ethoxy]ethyl and 2-acetaminoethyl.

6. A process as claimed in claim 2 in which R in the compound of formula (I) is 2,2-bis (hydroxymethyl)propionyloxy or 2,2,5-trimethyl[1.3-dioxane]-5-carbonyloxy.

7. A process as claimed in claim 6 in which the esterification step (c) is carried out using 2,2,5-trimethyl[1,3-dioxane]-5-carboxylic acid chloride or the 2,4,6-trichlorobenzoyl mixed anhydride of 2,2,5-trimethyl[1.3-dioxane]-5-carboxylic acid.

8. A process as claimed in claim 7 which is carried out in a solvent comprising methylene chloride.

9. A process as claimed in claim 7 or claim 8 which is carried out at a temperature of from about -50°C to about 25°C

10. A process as claimed in any one of claims 2 to 9 wherein the acid in step (d) is a dilute inorganic acid.

11. A process according to claim 10 wherein the acid is sulphuric, hydrochloric or phosphoric acid.

12. A process according to any one of claims 2 to 11 wherein the acid used in step (d) is from about 0.1N to about 3N.

13. A process according to any one of claims 2 to 11 wherein the acid used in step (d) is from about 0.2N to about 2N.

14. A process according to any one of claims 2 to 11 wherein the acid used in step (d) is about 0.5N.

15. A process according to any one of claims 2 to 14 wherein step (d) is carried out in a single phase aqueous acid/organic solvent system.

16. A process according to claim 15 wherein the organic solvent is acetone.

17. A process according to any one of claims 2 to 16 wherein step (d) is carried out at a temperature from about 25°C or below.

18. A process according to any one of claims 2 to 16, wherein step (d) is carried out at a temperature from about -5°C to about 10°C.

19. A process according to any one of claims 2 to 16 wherein step (d) is carried out at a temperature from about 0°C to about 5°C.

20. A process according to any one of claims 1 to 19 in which the 31-silyl ether prepared in step (b) has the formula IA: 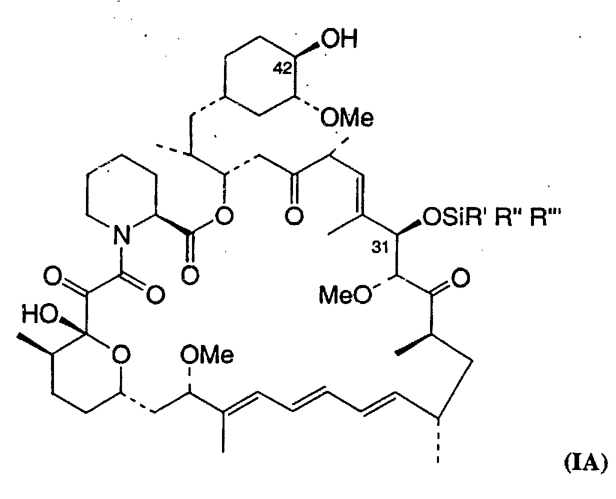 wherein R', R" and R"' are the same or different selected from alkyl of 1-6 carbon atoms, phenyl and benzyl.

21. A process according to any one of claims 1 to 20 wherein the silylating agent in step (a) is a tri-(C₁-C₆)alkylsilyl halide.

22. A process according to claim 21 wherein the silylating agent in step (a) is trimethylsilyl chloride.

23. A process as claimed in any one of claims 1 to 22 in which the silylation step (a) is carried out in an inert solvent in the presence of a suitable base.

24. A process according to claim 23 wherein the base is imidazole, 1-methylimidazole, triethylamine, or N, N-diisopropylethylamine.

25. A process according to any one of claims 1 to 24 wherein the acid in step (b) is a dilute inorganic acid.

26. A process according to claim 25 wherein the acid is sulfuric, hydrochloric or phosphoric acid.

27. A process according to any one of claims 1 to 26 wherein the acid used in step (b) is from about 0.08N to about 2.5N.

28. A process according to any one of claims 1 to 26 wherein the acid strength in step (b) is from about 0.1N to about 1N.

29. A process according to any one of claims 1 to 26, wherein the acid used in step (b) is about 0.5N.

30. A process according to any one of claims 1 to 29, wherein step (b) is carried out in a two phase aqueous acid/water-immiscible organic solvent system.

31. A process according to claim 30 wherein the water-immiscible organic solvent is ethyl acetate.

32. A process according to any one of claims 1 to 31 wherein step (b) is carried out at a temperature from about 25°C or below.

33. A process according to any one of claims 1 to 31 wherein step (b) is carried out at a temperature from about -5°C to about 10°C.

34. A process according to any one of claims 1 to 31 wherein step (b) is carried out at a temperature from about 0°C to about 5°C.

35. A process for preparing rapamycin 42-ester with 2,2-bis-(hydroxymethyl)propionic acid, which comprises:
(a) reacting rapamycin with a silylating agent to form rapamycin 31,42-bis-silyl ether;
(b) selectively hydrolyzing the 42-silyl ether in cold dilute acid to provide rapamycin 31-silyl ether;
(c) acylating the rapamycin 31-silyl ether with 2,2,5-trimethyl[1.3-dioxane]-5-carboxylic acid chloride or the 2,4,6-trichlorobenzoyl mixed anhydride of 2,2,5-trimethyl[1.3-dioxane]-5-carboxylic acid to give raparnycin 31-O-trimethylsil ether, 42-ester with 2.2,5-trimethyl[1.3-dioxane]-5-carboxylic acid;
and (d)
either
(i) selectively hydrolyzing the 31-silyl ether in cold dilute acid to provide rapamycin 42-ester with 2,2,5-trimethyl[1.3-dioxane]-5-carboxylic acid; followed by treating the rapamycin 42-ester with 2,2,5-trimethyl[1.3-dioxane]-5-carboxylic acid with dilute acid to provide 42-ester with 2,2-bis-(hydroxymethyl)propionic acid;
or
(ii) treating the rapamycin 31-O-trimethylsilyl ether, 42-ester with 2,2,5-trimethyl[1.3-dioxane]-5-carboxylic acid with dilute acid to provide 42-ester with 2,2-bis-(hydroxymethyl)propionic acid;

36. The process according to claim 35 wherein the acylation step (c) is carried out at a temperature less than 0°C.

37. A process for preparing 42-O-(2-hydroxy)ethyl-rapamycin which comprises:
(a) reacting rapamycin with a silylating agent to form rapamycin 31,42-bis-silyl ether;
(b) selectively hydrolyzing the 42-silyl ether in cold dilute acid to provide rapamycin 31-silyl ether;
(c) reacting the rapamycin 31-silyl ether with an ethylene glycol equivalent containing an acid labile hydroxyl protecting group protected on one terminus of the ethylene glycol equivalent and a leaving group suitable for alkylating a hydroxyl group as the other terminus of the ethylene glycol equivalent.
(d) hydrolysing the protecting groups on the 31-position and on the 42-hydroxyethyl position under mildly acidic conditions.

38. The process according to claim 37 wherein the silylating agent is a trialkylsilyl halide.

39. The process according to claim 38 wherein the silylating agent is chlorotrimethylsilane.

40. The process according to any one of claims 37 to 39 wherein the ethylene glycol equivalent is 2-(t-butyldimethylsilyl)oxyethyl triflate).

41. The process according to claim 40 wherein the acid used in steps (b) and (d) is sulfuric acid.

42. A compound which is a rapamycin 31-O-silyl ether.

43. A compound according to claim 42 which has the formula IA or IB: 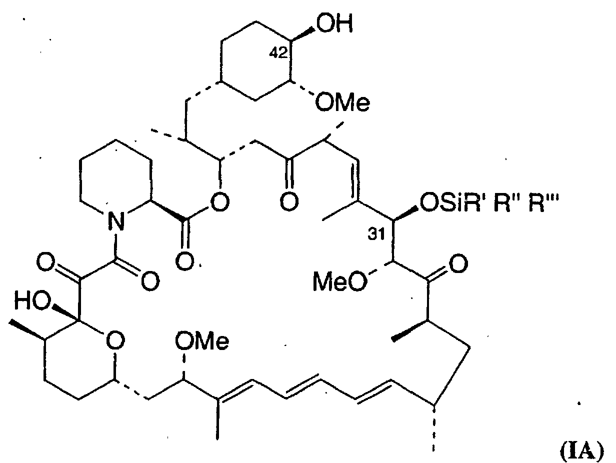 or 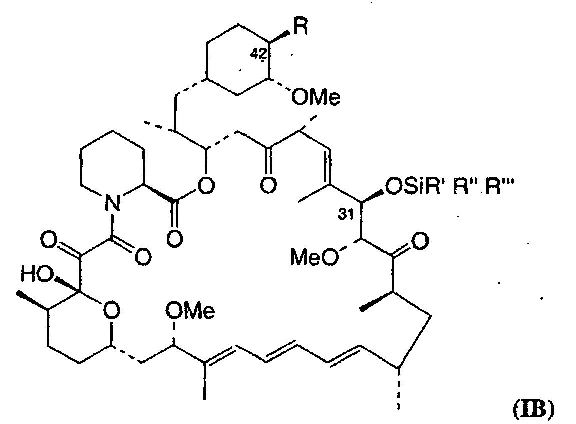 in which formulae,
R is an ester or ether group,
and R', R" and R"' are the same or different selected from alkyl of 1-6 carbon atoms, phenyl and benzyl.

44. A compound of claim 42 wherein R is as defined in claim 3.

45. A compound of claim 42 or claim 43 in which the 31-O-silyl ether is a tri-(C₁-C₆)alkylsilyl ether.

46. A compound of claim 42, which is rapamycin 31-O-trimethylsilyl ether.

47. A compound of claim 42 which has formula (D) 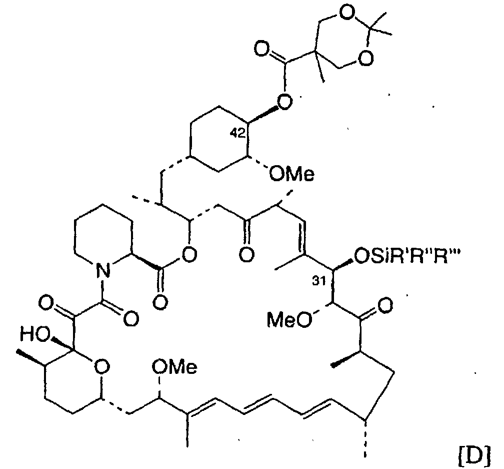 wherein R', R" and R"' are the same or different selected from alkyl of 1-6 carbon atoms, phenyl and benzyl.

48. A compound of claim 42 which is rapamycin 31-O-trimethylsilyl ether, 42-ester with 2,2,5-trimethyl[1.3-dioxane]-5-carboxylic acid.

49. A process for preparing a compound of formula (C): 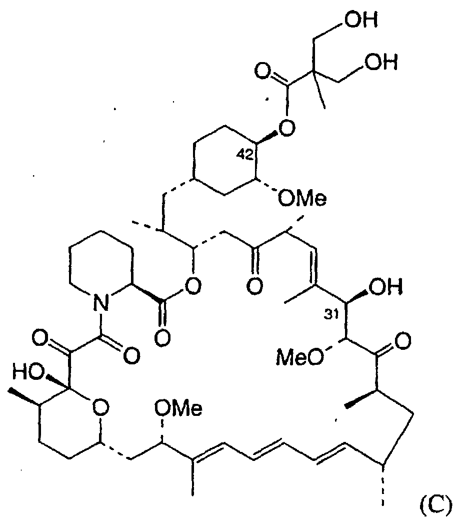 which comprises hydrolysing a compound of formula (B): 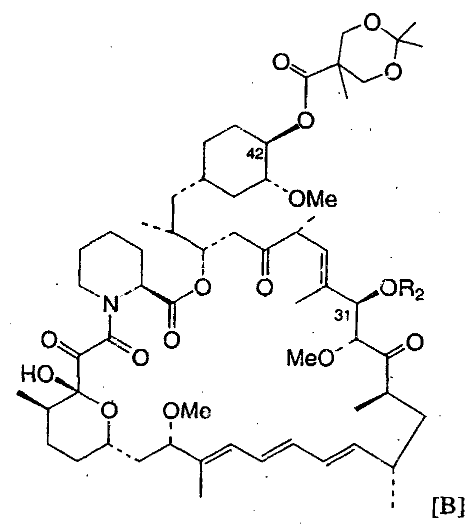 in which R₂ is hydrogen or -SiR'R"R"' wherein R', R" and R"' are the same or different selected from alkyl of 1-6 carbon atoms, phenyl and benzyl;
using dilute sulfuric acid.

50. A process as claimed in Claim 49 which is carried out using 1N to 3N sulfuric acid.

51. A process as claimed in Claim 49 or Claim 50 which is carried out at a temperature of -5°C to +10°C.

52. A process as claimed in any one of Claims 49 to 51 which is carried out in the presence of tetrahydrofuran solvent.

## Patentansprüche

1. Verfahren zum Herstellen von Rapamycin-31-silylether, welches umfasst:
(a) Umsetzen von Rapamycin mit einem Silylierungsmittel, um Rapamycin-31,42-bis-silylether zu bilden; und
(b) Hydrolysieren des 31,42-Bis-silylethers mit kalter, verdünnter Säure, um Rapamycin-31-silylether vorzusehen.

2. Verfahren zum Herstellen eines 42-Esters oder Ethers von Rapamycin mit der Struktur 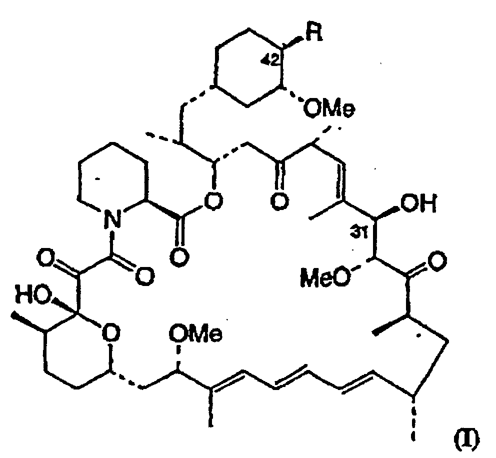 worin R einen Ester oder Ether darstellt, welches umfasst:
(a) Umsetzen von Rapamycin mit einem Silylierungsmittel, um Rapamycin-31,42-bis-silylether zu bilden;
(b) Hydrolysieren des 31,42-Bis-silylethers in kalter, verdünnter Säure, um Rapamycin-31-silylether vorzusehen;
(c) Umsetzen des Rapamycin-31-silylethers mit einem geeigneten Veresterungs- oder Veretherungs-Reagenz, um Rapamycin-31-silylether-42-ester oder -ether zu bilden; und
(d) Hydrolysieren des 31-Silylethers in kalter, verdünnter Säure, um den gewünschten Rapamycin-42-ester oder -ether vorzusehen; falls gewünscht mit aufeinanderfolgendem oder gleichzeitigem Entfernen jeder anderen vorhandenen Schutzgruppe.

3. Verfahren, wie in Anspruch 2 beansprucht, wobei R in der Verbindung der Formel (I) ausgewählt wird aus:
(i) -O-C=O.CR⁷R⁸R⁹; (also Estern) worin:
R⁷ Wasserstoff, Alkyl mit 1-6 Kohlenstoffatomen, Alkenyl mit 2-7 Kohlenstoffatomen, Alkinyl mit 2-7 Kohlenstoffatomen, -(CR³R⁴)_{f}OR¹⁰, -CF₃, -F oder -CO₂R¹¹ darstellt;
R⁸ und R⁹ jeweils unabhängig Wasserstoff, Alkyl mit 1-6 Kohlenstoffatomen oder (CR³R⁴)_{f}OR¹⁰ darstellen, oder R⁸ und R⁹ zusammengenommen werden können, um X zu bilden;
R¹⁰ Wasserstoff, Alkyl mit 1-6 Kohlenstoffatomen, Alkenyl mit 2-7 Kohlenstoffatomen, Alkinyl mit 2-7 Kohlenstoffatomen, Triphenylmethyl, Benzyl, Alkoxymethyl mit 2-7 Kohlenstoffatomen, Chlorethyl oder Tetrahydropyranyl darstellt;
X für 5-(2,2-Di-(alkyl mit 1-6 Kohlenstoffatomen))[1,3]-dioxanyl, 5-(2-Spiro(cycloalkyl mit 3-8 Kohlenstoffatomen))[1,3]-dioxanyl, 4-(2,2-Di-(alkyl mit 1-6 Kohlenstoffatomen))[1,3]-dioxanyl, 4-(2-Spiro(cycloalkyl mit 3-8 Kohlenstoffatomen))[1,3]dioxanyl, 4-(2,2-Di-(alkyl mit 1-6 Kohlenstoffatomen))[1,3]dioxalanyl oder 4-(2-Spiro(cycloalkyl mit 3-8 Kohlenstoffatomen))[1,3]dioxalanyl steht;
R³ und R⁴ jeweils unabhängig Wasserstoff, Alkyl mit 1-6 Kohlenstoffatomen, Alkenyl mit 2-7 Kohlenstoffatomen, Alkinyl mit 2-7 Kohlenstoffatomen, Trifluormethyl oder -F darstellen; und
f = 0-6; unter der Bedingung, dass R mindestens eine -(CR³R⁴)_{f}OR¹⁰- oder X-Gruppe enthält;
oder
(ii) -OR¹ (also Ether), worin R¹ Alkyl, Thioalkyl, Arylalkyl, Hydroxyalkyl, Dihydroxyalkyl, Hydroxyalkylarylalkyl, Dihydroxyalkylarylalkyl, Alkoxyalkyl, Acyloxyalkyl, Aminoalkyl, Alkylaminoalkyl, Alkoxycarbonylaminoalkyl, Acylaminoalkyl, Arylsulfonamidoalkyl, Allyl, Dihydroxyalkylallyl und Dioxolanylallyl, Carbalkoxyalkyl darstellt; worin "Alk-" oder "Alkyl" C₁₋₆-Alkyl bezeichnet, verzweigt oder linear, vorzugsweise C₁₋₃-Alkyl, worin die Kohlenstoffkette gegebenenfalls durch eine Ether-(O)-Bindung unterbrochen sein kann; "Acyl" für Alkylcarbonyl steht und "Aryl" 6-10 Kohlenstoffatome hat.

4. Verfahren wie in Anspruch 3 beansprucht, wobei R¹ ausgewählt wird aus Hydroxyalkyl, Hydroxyalkoxyalkyl, Acylaminoalkyl und Aminoalkyl.

5. Verfahren wie in Anspruch 4 beansprucht, wobei R¹ ausgewählt wird aus 2-Hydroxyethyl; 3-Hydroxypropyl; 2-[(2-Hydroxy)-ethoxy]ethyl und 2-Acetaminoethyl.

6. Verfahren, wie in Anspruch 2 beansprucht, wobei R in der Verbindung der Formel (I) für 2,2-Bis(hydroxymethyl)-propionyloxy oder 2,2,5-Trimethyl[1,3-dioxan]-5-carbonyloxy steht.

7. Verfahren wie in Anspruch 6 beansprucht, wobei der Veresterungsschritt (c) unter Verwendung von 2,2,5-Trimethyl[1,3-dioxan]-5-carbonsäurechlorid oder dem 2,4,6-Trichlorbenzoyl gemischten Anhydrid von 2,2,5-Trimethyl[1,3-dioxan]-5-carbonsäure durchgeführt wird.

8. Verfahren wie in Anspruch 7 beansprucht, welches in einem Methylenchlorid umfassenden Lösungsmittel durchgeführt wird.

9. Verfahren, wie in Anspruch 7 oder Anspruch 8 beansprucht, welches bei einer Temperatur von etwa -50°C bis etwa 25°C durchgeführt wird.

10. Verfahren, wie in einem der Ansprüche 2 bis 9 beansprucht, wobei die Säure in Schritt (d) eine verdünnte anorganische Säure ist.

11. Verfahren gemäß Anspruch 10, wobei die Säure Schwefel-, Salz- oder Phosphorsäure ist.

12. Verfahren gemäß einem der Ansprüche 2 bis 11, wobei die in Schritt (d) verwendete Säure von etwa 0,1N bis etwa 3N beträgt.

13. Verfahren gemäß einem der Ansprüche 2 bis 11, wobei die in Schritt (d) verwendete Säure von etwa 0,2N bis etwa 2N beträgt.

14. Verfahren gemäß einem der Ansprüche 2 bis 11, wobei die in Schritt (d) verwendete Säure etwa 0,5N beträgt.

15. Verfahren gemäß einem der Ansprüche 2 bis 14, wobei Schritt (d) in einem einphasigen wässerige Säure/organischen Lösungsmittel System durchgeführt wird.

16. Verfahren gemäß Anspruch 15, wobei das organische Lösungsmittel Aceton ist.

17. Verfahren gemäß einem der Ansprüche 2 bis 16, wobei Schritt (d) bei einer Temperatur von etwa 25°C oder darunter durchgeführt wird.

18. Verfahren gemäß einem der Ansprüche 2 bis 16, wobei Schritt (d) bei einer Temperatur von etwa -5°C bis etwa 10°C durchgeführt wird.

19. Verfahren gemäß einem der Ansprüche 2 bis 16, wobei Schritt (d) bei einer Temperatur von etwa 0°C bis etwa 5°C durchgeführt wird.

20. Verfahren gemäß einem der Ansprüche 1 bis 19, wobei der in Schritt (b) hergestellte 31-Silylether die Formel IA aufweist: 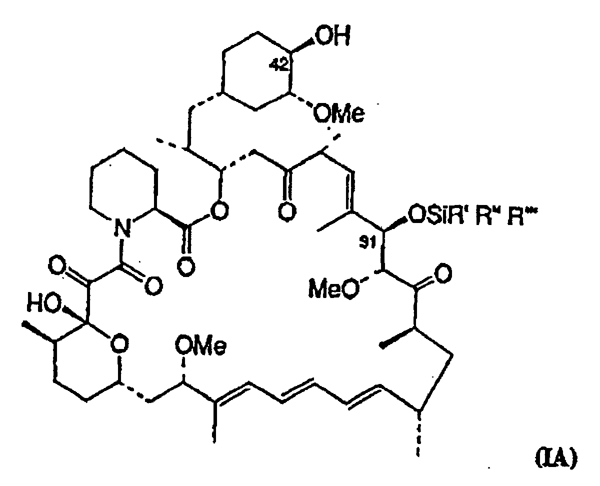 worin R', R'' und R''' gleich oder unterschiedlich sind, ausgewählt aus Alkyl mit 1-6 Kohlenstoffatomen, Phenyl und Benzyl.

21. Verfahren gemäß einem der Ansprüche 1 bis 20, wobei das Silylierungsmittel in Schritt (a) ein Tri-(C₁-C₆)alkylsilylhalogenid ist.

22. Verfahren gemäß Anspruch 21, wobei das Silylierungsmittel in Schritt (a) Trimethylsilylchlorid ist.

23. Verfahren wie in einem der Ansprüche 1 bis 22 beansprucht, wobei der Silylierungsschritt (a) in einem inerten Lösungsmittel in Gegenwart einer geeigneten Base durchgeführt wird.

24. Verfahren gemäß Anspruch 23, wobei die Base Imidazol, 1-Methylimidazol, Triethylamin oder N,N-Diisopropylethylamin ist.

25. Verfahren gemäß einem der Ansprüche 1 bis 24, wobei die Säure in Schritt (b) eine verdünnte organische Säure ist.

26. Verfahren gemäß Anspruch 25, wobei die Säure Schwefel-, Salz- oder Phosphorsäure ist.

27. Verfahren gemäß einem der Ansprüche 1 bis 26, wobei die in Schritt (b) verwendete Säure von etwa 0,08N bis etwa 2,5N beträgt.

28. Verfahren gemäß einem der Ansprüche 1 bis 26, wobei die Säurestärke in Schritt (b) von etwa 0,1N bis etwa 1N beträgt.

29. Verfahren gemäß einem der Ansprüche 1 bis 26, wobei die in Schritt (b) verwendete Säure etwa 0,5N beträgt.

30. Verfahren gemäß einem der Ansprüche 1 bis 29, wobei Schritt (b) in einem zweiphasigen wässerige Säure/mit Wasser nicht mischbaren organischen Lösungsmittel System durchgeführt wird.

31. Verfahren gemäß Anspruch 30, wobei das mit Wasser nicht mischbare organische Lösungsmittel Ethylacetat ist.

32. Verfahren gemäß einem der Ansprüche 1 bis 31, wobei Schritt (b) bei einer Temperatur von etwa 25°C oder darunter durchgeführt wird.

33. Verfahren gemäß einem der Ansprüche 1 bis 31, wobei Schritt (b) bei einer Temperatur von etwa -5°C bis etwa 10°C durchgeführt wird.

34. Verfahren gemäß einem der Ansprüche 1 bis 31, wobei Schritt (b) bei einer Temperatur von etwa 0°C bis etwa 5°C durchgeführt wird.

35. Verfahren zum Herstellen von Rapamycin-42-ester mit 2,2-Bis-(hydroxy-methyl)propionsäure, welches umfasst:
(a) Umsetzen von Rapamycin mit einem Silylierungsmittel, um Rapamycin-31,42-bis-silylether zu bilden;
(b) Selektives Hydrolysieren des 42-Silylethers in kalter verdünnter Säure, um Rapamycin-31-silylether vorzusehen;
(c) Acylieren des Rapamycin-31-silylethers mit 2,2,5-Trimethyl[1,3-dioxan]-5-carbonsäurechlorid oder dem 2,4,6-Trichlorbenzoyl gemischten Anhydrid von 2,2,5-Trimethyl-[1,3-dioxan]-5-carbonsäure, um Rapamycin-31-O-trimethylsilylether, 42-Ester mit 2,2,5-Trimethyl[1,3-dioxan]-5-carbonsäure zu ergeben; und
(d) entweder
(i) selektives Hydrolysieren des 31-Silylethers in kalter verdünnter Säure, um Rapamycin-42-ester mit 2,2,5-Trimethyl[1,3-dioxan]-5-carbonsäure vorzusehen; gefolgt von Behandeln des Rapamycin-42-esters mit 2,2,5-Trimethyl[1,3-dioxan]-5-carbonsäure mit verdünnter Säure, um 42-Ester mit 2,2-Bis-(hydroxymethyl)-propionsäure vorzusehen; oder
(ii) Behandeln von Rapamycin-31-O-trimethylsilylether, 42-Ester mit 2,2,5-Trimethyl[1,3-dioxan]-5-carbonsäure mit verdünnter Säure, um 42-Ester mit 2,2-Bis-(hydroxymethyl)-propionsäure vorzusehen.

36. Verfahren gemäß Anspruch 35, wobei der Acylierungsschritt (c) bei einer Temperatur von weniger als 0°C durchgeführt wird.

37. Verfahren zum Herstellen von 42-O-(2-Hydroxy)ethyl-rapamycin, welches umfasst:
(a) Umsetzen von Rapamycin mit einem Silylierungsmittel, um Rapamycin-31,42-bis-silylether zu bilden;
(b) Selektives Hydrolysieren des 42-Silylethers in kalter verdünnter Säure, um Rapamycin-31-silylether vorzusehen;
(c) Umsetzen des Rapamycin-31-silylethers mit einem Ethylenglycol-Äquivalent, welches eine Säure-instabile Hydroxyl-Schutzgruppe enthält, geschützt an einem Endpunkt des Ethylenglycol-Äquivalents, und eine zum Alkylieren einer Hydroxylgruppe geeignete Abgangsgruppe als anderen Endpunkt des Ethylenglycol-Äquivalents.
(d) Hydrolysieren der Schutzgruppen an der 31-Position und an der 42-Hydroxyethylposition unter leicht sauren Bedingungen.

38. Verfahren gemäß Anspruch 37, wobei das Silylierungsmittel ein Trialkylsilylhalogenid ist.

39. Verfahren gemäß Anspruch 38, wobei das Silylierungsmittel Chlortrimethylsilan ist.

40. Verfahren gemäß einem der Ansprüche 37 bis 39, wobei das Ethylenglycol-Äquivalent 2-(t-Butyldimethylsilyl)oxyethyltriflat ist.

41. Verfahren gemäß Anspruch 40, wobei die in Schritten (b) und (d) verwendete Säure Schwefelsäure ist.

42. Verbindung, welche Rapamycin-31-O-silylether ist.

43. Verbindung gemäß Anspruch 42, welche die Formel IA oder IB aufweist: 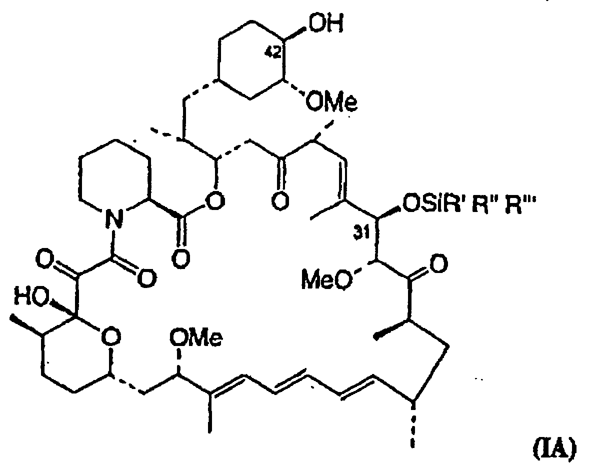 oder 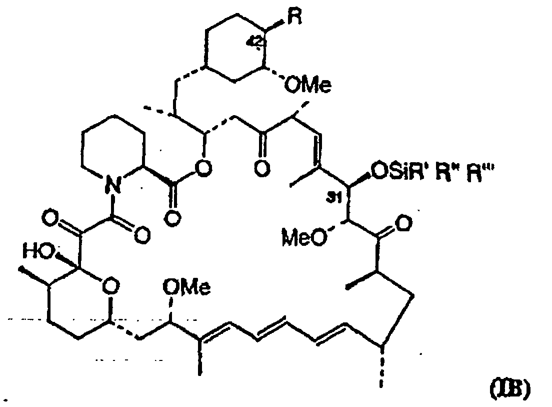 in welchen Formeln
R einer Ester- oder Ethergruppe darstellt, und
R', R'' und R''' gleich oder unterschiedlich sind, ausgewählt aus Alkyl mit 1-6 Kohlenstoffatomen, Phenyl und Benzyl.

44. Verbindung nach Anspruch 42, worin R wie in Anspruch 3 definiert ist.

45. Verbindung nach Anspruch 42 oder Anspruch 43, worin der 31-O-Silylether ein Tri-(C₁-C₆)-alkylsilylether ist.

46. Verbindung nach Anspruch 42, welcher Rapamycin-31-O-trimethylsilylether ist.

47. Verbindung nach Anspruch 42, welche die Formel (D) aufweist: 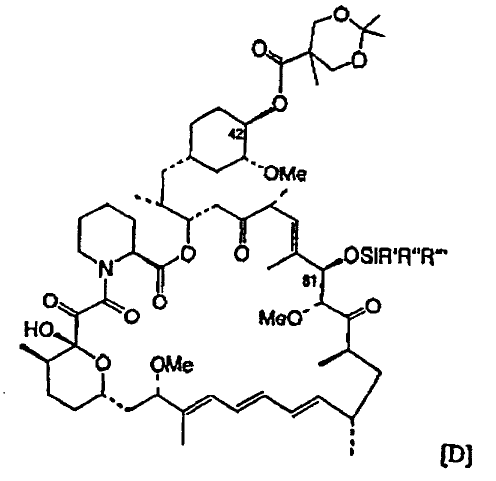 worin R', R'' und R''' gleich oder unterschiedlich sind, ausgewählt aus Alkyl mit 1-6 Kohlenstoffatomen, Phenyl und Benzyl.

48. Verbindung nach Anspruch 42, welche Rapamycin-31-O-trimethylsilylether, 42-Ester mit 2,2,5-Trimethyl[1,3-dioxan]-5-carbonsäure ist.

49. Verfahren zum Herstellen einer Verbindung der Formel (C): 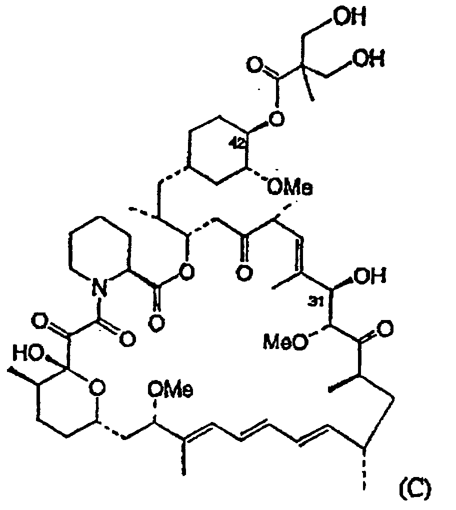 welches Hydrolysieren einer Verbindung der Formel (B) : 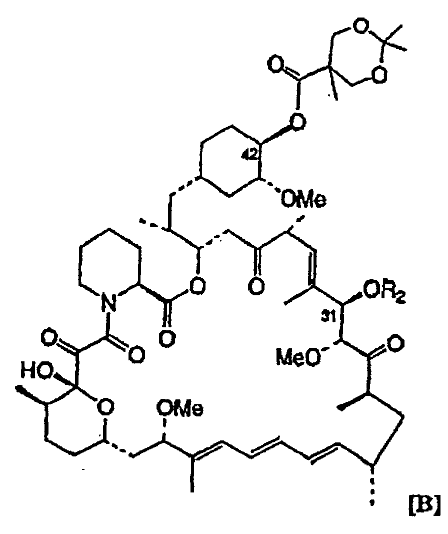 umfasst, worin R₂ für Wasserstoff oder -SiR'R''R''' steht, worin R', R'' und R''' gleich oder unterschiedlich sind, ausgewählt aus Alkyl mit 1-6 Kohlenstoffatomen, Phenyl und Benzyl; unter Verwendung von verdünnter Schwefelsäure.

50. Verfahren, wie in Anspruch 49 beansprucht, welches unter Verwendung von 1N bis 3N Schwefelsäure durchgeführt wird.

51. Verfahren, wie in Anspruch 49 oder Anspruch 50 beansprucht, welches bei einer Temperatur von -5°C bis +10°C durchgeführt wird.

52. Verfahren, wie in einem der Ansprüche 49 bis 51 beansprucht, welches in Gegenwart von Tetrahydrofuran-Lösungsmittel durchgeführt wird.

## Revendications

1. Procédé de préparation de 31-silyléther de la rapamycine, qui comprend :
(a) la réaction de la rapamycine avec un agent silylant pour former un 31, 42-bissilyléther de la rapamycine; et
(b) l'hydrolyse du 31, 42-bissilyléther avec un acide dilué froid pour procurer un 31-silyléther de la rapamycine.

2. Procédé de préparation d'un 42-ester ou éther de la rapamycine présentant la structure 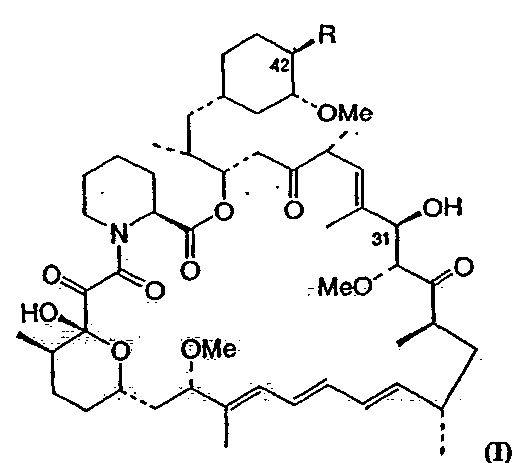 dans laquelle R est un ester ou un éther, qui comprend :
(a) la réaction de la rapamycine avec un agent silylant pour former un 31, 42-bissilyléther de la rapamycine; et
(b) l'hydrolyse du 31, 42-bissilyléther dans un acide dilué froid pour procurer un 31-silyléther de la rapamycine;
(c) la réaction du 31-silyléther de la rapamycine avec un réactif approprié d'estérification ou d'étherification pour former un 31-silyléther, 42-ester ou éther de la rapamycine; et
(d) l'hydrolyse du 31-silyléther dans un acide dilué froid pour donner le 42-ester ou éther souhaité de la rapamycine; si souhaité une élimination séquentielle ou simultanée d'un quelconque autre groupement protecteur présent.

3. Procédé suivant la revendication 2, dans lequel R dans le composé de formule (I) est sélectionné parmi :
soit
(i) -O-C=O.CR⁷R⁸R⁹; (c'est-à-dire des esters) dans lesquels :
R⁷ est un hydrogène, un alkyle de 1-6 atomes de carbone, un alcényle de 2-7 atomes de carbone, un alcynyle de 2-7 atomes de carbone, -(CR³R⁴)_{f}OR¹⁰, -CF₃, -F, ou -CO₂R¹¹;
R⁸ et R⁹ sont chacun, indépendamment, un hydrogène, un alkyle de 1-6 atomes de carbone, ou -(CR³R⁴)_{f}OR¹⁰; ou R⁸ et R⁹ peuvent être pris ensemble pour former X;
R¹⁰ est un hydrogène, un alkyle de 1-6 atomes de carbone, un alcényle de 2-7 atomes de carbone, un alcynyle de 2-7 atomes de carbone, un triphénylméthyle, un benzyle, un alcoxyméthyle de 2-7 atomes de carbone, un chloroéthyle, ou un tétrahydropyrannyle;
X est un 5-(2,2-di-(alkyle de 1-6 atomes de carbone))[1,3]dioxannyle, un 5-(2-spiro(cycloalkyle de 3-8 atomes de carbone))[1,3]dioxannyle, un 4-(2,2-di(alkyle de 1-6 atomes de carbone))[1,3]dioxannyle, un 4-(2-spiro(cycloalkyle de 3-8 atomes de carbone))[1,3]dioxannyle, un 4-(2,2-di-(alkyle de 1-6 atomes de carbone))-[1,3]dioxalanyle, ou un 4-(2-spiro(cycloalkyle de 3-8 atomes de carbone))[1,3]dioxalanyle;
R³ et R⁴ sont chacun, indépendamment, un hydrogène, un alkyle de 1-6 atomes de carbone, un alcényle de 2-7 atomes de carbone, un alcynyle de 2-7 atomes de carbone, un trifluorométhyle, ou -F;
et
f=0-6; à condition que R contienne au moins un groupement -(CR³R⁴)_{f}OR¹⁰ ou X;
soit
(ii) -OR¹ (c'est-à-dire, des éthers) dans lesquels R¹ est un alkyle, un thioalkyle, un arylalkyle, un hydroxyalkyle, un dihydroxyalkyle, un hydroxyalkylarylalkyle, un dihydroxyalkylarylalkyle, un alcoxyalkyle, un acyloxyalkyle, un aminoalkyle, un alkylaminoalkyle, un alcoxycarbonylaminoalkyle, un acylaminoalkyle, un arylsulfonamidoalkyle, un allyle, un dihydroxyalkylallyle et un dioxolanylallyle, un carbalcoxyalkyle; dans lesquels "alk-" ou "alkyl" fait référence à un alkyle en C₁-C₆, ramifié ou linéaire, de préférence un alkyle en C₁-C₃, dans lequel la chaîne carbonée peut être facultativement interrompue par un lien éther (-O-); "acyle" représente un alkylcarbonyle et "aryl" présente 6-10 atomes de carbone.

4. Procédé suivant la revendication 3, dans lequel R¹ est sélectionné parmi un hydroxyalkyle, un hydroxyalcoxyalkyle, un acylaminoalkyle et un aminoalkyle.

5. Procédé suivant la revendication 4, dans lequel R¹ est sélectionné parmi un 2-hydroxyéthyle; un 3-hydroxypropyle; un 2-[(2-hydroxy)éthoxy]éthyle et un 2-acétaminoéthyle.

6. Procédé suivant la revendication 2, dans lequel R dans le composé de formule (I) est un 2,2-bis(hydroxyméthyl)propionyloxy ou un 2,2,5-triméthyl[1,3-dioxanne]-5-carbonyloxy.

7. Procédé suivant 1a revendication 6, dans lequel l'étape d'estérification (c) est réalisée en utilisant du chlorure d'acide 2,2,5-triméthyl-[1,3-dioxanne]-5-carboxylique ou l'anhydride 2,4,6-trichlorobenzoyle mixte de l'acide 2,2,5-triméthyl[1,3-dioxanne]-5-carboxylique.

8. Procédé suivant la revendication 7, qui est réalisé dans un solvant comprenant du chlorure de méthylène.

9. Procédé suivant la revendication 7 ou la revendication 8, qui est réalisé à une température d'environ -50°C à environ 25°C.

10. Procédé suivant l'une quelconque des revendications 2 à 9, dans lequel l'acide à l'étape (d) est un acide inorganique dilué.

11. Procédé suivant la revendication 10, dans lequel l'acide est de l'acide sulfurique, chlorhydrique ou phosphorique.

12. Procédé suivant l'une quelconque des revendications 2 à 11, dans lequel l'acide utilisé à l'étape (d) est d'environ 0,1 N à environ 3 N.

13. Procédé suivant l'une quelconque des revendications 2 à 11, dans lequel l'acide utilisé à l'étape (d) est d'environ 0,2 N à environ 2 N.

14. Procédé suivant l'une quelconque des revendications 2 à 11, dans lequel l'acide utilisé à l'étape (d) est d'environ 0,5 N.

15. Procédé suivant l'une quelconque des revendications 2 à 14, dans lequel l'étape (d) est réalisée dans un système de solvants acide aqueux/organique en une seule phase.

16. Procédé suivant la revendication 15, dans lequel le solvant organique est de l'acétone.

17. Procédé suivant l'une quelconque des revendications 2 à 16, dans lequel l'étape (d) est réalisée à une température d'environ 25°C ou inférieure.

18. Procédé suivant l'une quelconque des revendications 2 à 16, dans lequel l'étape (d) est réalisée à une température d'environ -5°C à environ 10°C.

19. Procédé suivant l'une quelconque des revendications 2 à 16, dans lequel l'étape (d) est réalisée à une température d'environ 0°C à environ 5°C.

20. Procédé suivant l'une quelconque des revendications 1 à 19, dans lequel le 31-silyléther préparé à l'étape (b) présente la formule IA : 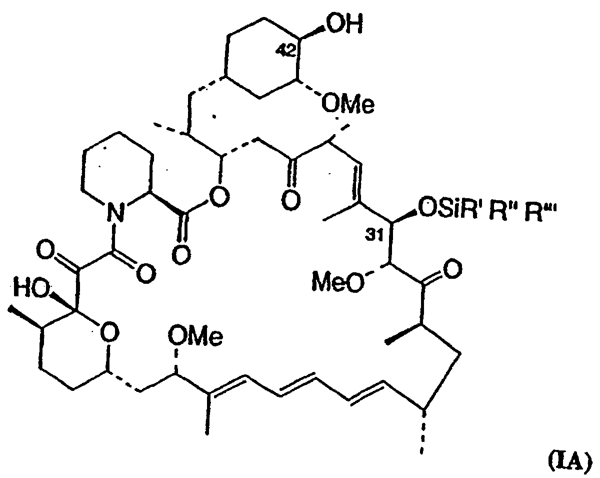 dans laquelle R', R'' et R''', identiques ou différents, sont sélectionnés parmi un alkyle de 1-6 atomes de carbone, un phényle et un benzyle.

21. Procédé suivant l'une quelconque des revendications 1 à 20, dans lequel l'agent silylant à l'étape (a) est un halogénure de tri(alkyl en C₁-C₆)silyle.

22. Procédé suivant la revendication 21, dans lequel l'agent silylant à l'étape (a) est du chlorure de triméthylsilyle.

23. Procédé suivant l'une quelconque des revendications 1 à 22, dans lequel l'étape de silylation (a) est réalisée dans un solvant inerte en présence d'une base appropriée.

24. Procédé suivant la revendication 23, dans lequel la base est de l'imidazole, du 1-méthylimidazole, de la triéthylamine, ou de la N,N-di-i-propyléthylamine.

25. Procédé suivant l'une quelconque des revendications 1 à 24, dans lequel l'acide à l'étape (b) est un acide inorganique dilué.

26. Procédé suivant la revendication 25, dans lequel l'acide est de l'acide sulfurique, chlorhydrique ou phosphorique.

27. Procédé suivant l'une quelconque des revendications 1 à 26, dans lequel l'acide utilisé à l'étape (b) est d'environ 0,08 N à environ 2,5 N.

28. Procédé suivant l'une quelconque des revendications 1 à 26, dans lequel la concentration de l'acide à l'étape (b) est d'environ 0,1 N à environ 1 N.

29. Procédé suivant l'une quelconque des revendications 1 à 26, dans lequel l'acide utilisé à l'étape (b) est d'environ 0,5 N.

30. Procédé suivant l'une quelconque des revendications 1 à 29, dans lequel l'étape (b) est réalisée dans un système de solvants à deux phases, acide aqueux/organique non miscible à l'eau.

31. Procédé suivant la revendication 30, dans lequel le solvant organique non miscible à l'eau est de l'acétate d'éthyle.

32. Procédé suivant l'une quelconque des revendications 1 à 31, dans lequel l'étape (b) est réalisée à une température d'environ 25°C ou inférieure.

33. Procédé suivant l'une quelconque des revendications 1 à 31, dans lequel l'étape (b) est réalisée à une température d'environ -5°C à environ 10°C.

34. Procédé suivant l'une quelconque des revendications 1 à 31, dans lequel l'étape (b) est réalisée à une température d'environ 0°C à environ 5°C.

35. Procédé de préparation d'un 42-ester de la rapamycine avec de l'acide 2,2-bis(hydroxyméthyl)-propionique, qui comprend :
(a) la réaction de la rapamycine avec un agent silylant pour former un 31, 42-bissilyléther de la rapamycine;
(b) l'hydrolyse sélective du 42-silyléther dans de l'acide dilué froid pour procurer un 31-silyléther de la rapamycine;
(c) l'acylation du 31-silyléther de la rapamycine avec du chlorure d'acide 2,2,5-triméthyl[1,3-dioxanne]-5-carboxylique ou de l'anhydride 2,4,6-trichlorobenzoyle mixte de l'acide 2,2,5-triméthyl[1,3-dioxanne]-5-carboxylique pour donner le 31-O-triméthylsilyléther de la rapamycine, 42-ester avec l'acide 2,2,5-triméthyl[1,3-dioxanne]-5-carboxylique;
et (d)
soit
(i) l'hydrolyse sélective du 31-silyléther dans de l'acide dilué froid pour donner le 42-ester de la rapamycine avec l'acide 2,2,5-triméthyl-[1,3-dioxanne]-5-carboxylique; suivie par un traitement du 42-ester de la rapamycine avec l'acide 2,2,5-triméthyl[1,3-dioxanne]-5-carboxylique avec de l'acide dilué pour donner le 42-ester avec l'acide 2,2-bis(hydroxyméthyl)propionique;
soit
(ii) le traitement du 31-O-triméthylsilyléther de la rapamycine, 42-ester avec l'acide 2,2,5-triméthyl[1,3-dioxanne]-5-carboxylique avec de l'acide dilué pour donner un 42-ester avec l'acide 2,2-bis(hydroxyméthyl)propionique;

36. Procédé suivant la revendication 35, dans lequel l'étape d'acylation (c) est réalisée à une température inférieure à 0°C.

37. Procédé de préparation de la 42-O-(2-hydroxy)éthylrapamycine qui comprend :
(a) la réaction de la rapamycine avec un agent silylant pour former un 31, 42-bissilyléther de la rapamycine;
(b) l'hydrolyse sélective du 42-silyléther dans de l'acide dilué froid pour donner un 31-silyléther de la rapamycine;
(c) la réaction du 31-silyléther de la rapamycine avec un équivalent d'éthylèneglycol contenant un groupement protecteur d'hydroxyle labile en milieu acide protégé à une extrémité de l'équivalent d'éthylèneglycol et un groupement partant approprié pour une alkylation d'un groupement hydroxyle à l'autre extrémité de l'équivalent d'éthylèneglycol.
(d) l'hydrolyse des groupements protecteurs sur la position 31 et sur la position 42-hydroxyéthyle dans des conditions légèrement acides.

38. Procédé suivant la revendication 37, dans lequel l'agent silylant est un halogénure de trialkylsilyle.

39. Procédé suivant la revendication 38, dans lequel l'agent silylant est du chlorotriméthylsilane.

40. Procédé suivant l'une quelconque des revendications 37 à 39, dans lequel l'équivalent d'éthylèneglycol est dû 2-(t-butyldiméthylsilyl)oxy-éthyltriflate).

41. Procédé suivant la revendication 40, dans lequel l'acide utilisé aux étapes (b) et (d) est de l'acide sulfurique.

42. Composé qui est un 31-O-silyléther de la rapamycine.

43. Composé suivant la revendication 42 qui présente la formule IA ou IB : 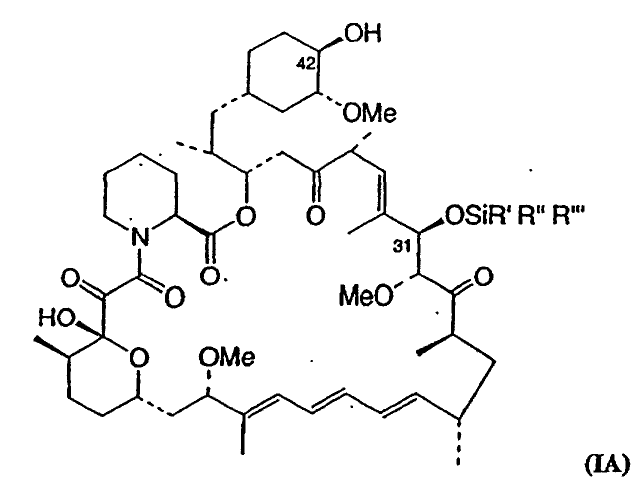 ou 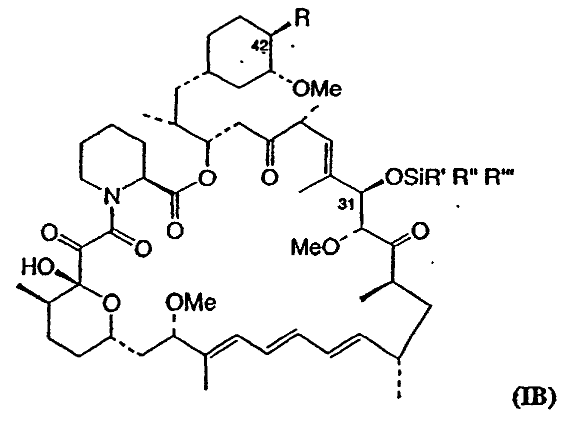 formules dans lesquelles,
R est un groupement ester ou éther,
et R', R'' et R''', identiques ou différents, sont sélectionnés parmi un alkyle de 1-6 atomes de carbone, un phényle et un benzyle.

44. Composé suivant la revendication 42, dans lequel R est comme défini à la revendication 3.

45. Composé suivant la revendication 42 ou la revendication 43, dans lequel le 31-O-silyléther est un tri-(alkyle en C₁-C₆)silyléther.

46. Composé suivant la revendication 42, qui est le 31-O-triméthylsilyléther de la rapamycine.

47. Composé suivant la revendication 42 qui présente la formule (D) 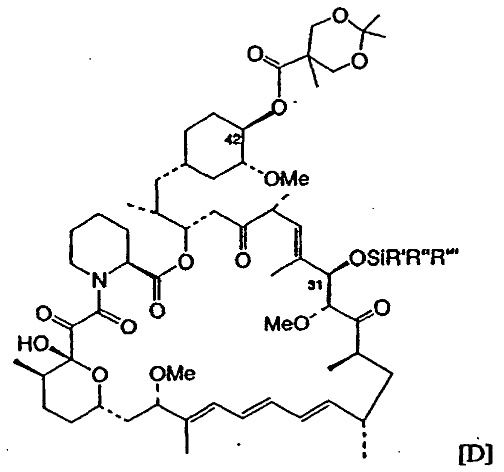 dans laquelle R', R'' et R''', identiques ou différents, sont sélectionnés parmi un alkyle de 1-6 atomes de carbone, un phényle et un benzyle.

48. Composé suivant la revendication 42 qui est le 31-O-triméthylsilyléther de la rapamycine, 42-ester avec l'acide 2,2,5-triméthyl[1,3-dioxanne]-5-carboxylique.

49. Procédé de préparation d'un composé de formule (C) : 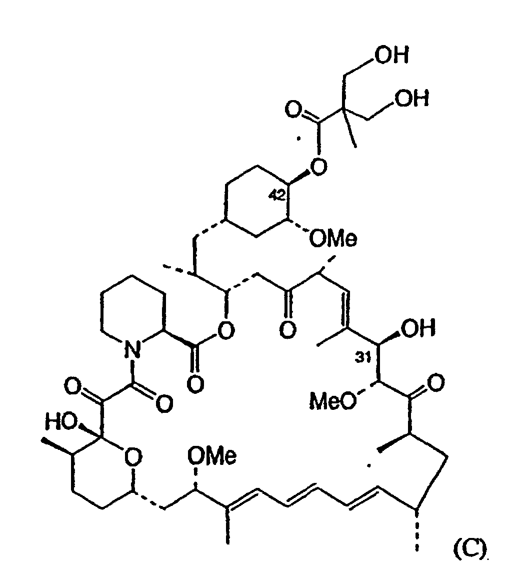 qui comprend l'hydrolyse d'un composé de formule (B) : 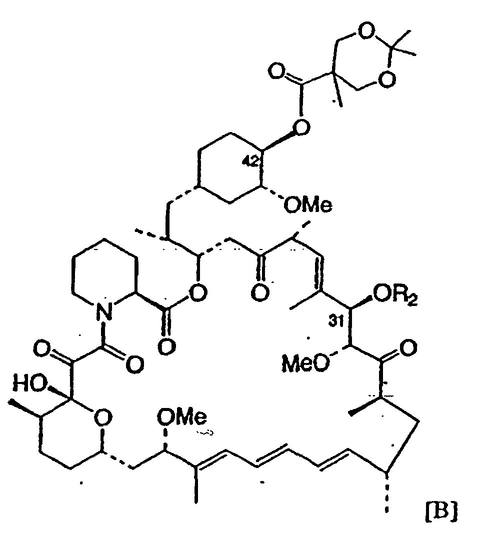 dans laquelle R₂ est un hydrogène ou -SiR'R''R'" dans laquelle R', R" et R''', identiques ou différents, sont sélectionnés parmi un alkyle de 1-6 atomes de carbone, un phényle et un benzyle; en utilisant de l'acide sulfurique dilué.

50. Procédé suivant la revendication 49, qui est réalisé en utilisant de l'acide sulfurique 1 N à 3 N.

51. Procédé suivant la revendication 49 ou la revendication 50, qui est réalisé à une température de -5°C à 10°C.

52. Procédé suivant l'une quelconque des revendications 49 à 51, qui est réalisé en présence de solvant tétrahydrofuranne.
